(19) Europäisches Patentamt
European Patent Office
Office européen des brevets

(11) EP 3 985 103 A1

(12) # EUROPEAN PATENT APPLICATION
published in accordance with Art. 153(4) EPC

(43) Date of publication:
**20.04.2022 Bulletin 2022/16**

(21) Application number: **19933188.5**

(22) Date of filing: **14.06.2019**

(51) International Patent Classification (IPC):
***C12N 5/073*** *(2010.01)* ***C12N 15/09*** *(2006.01)*

(52) Cooperative Patent Classification (CPC):
**C12N 15/09**

(86) International application number:
**PCT/JP2019/023725**

(87) International publication number:
**WO 2020/250438 (17.12.2020 Gazette 2020/51)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Tohoku University**
**Sendai-shi, Miyagi 980-8577 (JP)**

(72) Inventors:
- **ARIMA, Takahiro**
  **Sendai-shi, Miyagi 980-8577 (JP)**
- **KOBAYASH,I Norio**
  **Sendai-shi, Miyagi 980-8577 (JP)**
- **OKAE, Hiroaki**
  **Sendai-shi, Miyagi 980-8577 (JP)**

(74) Representative: **Cabinet Becker et Associés**
**25, rue Louis le Grand**
**75002 Paris (FR)**

(54) **CELLS CAPABLE OF DIFFERENTIATING INTO PLACENTA-CONSTITUTING CELLS, AND METHOD FOR PRODUCING SAME**

(57) A cell derived from a pluripotent stem cell and that is capable of differentiating into a placenta-forming cell, and that is negative for BRDT, and positive for TP63. A method for producing a cell capable of differentiating into a placenta-forming cell, the method including the steps of: (a) culturing a pluripotent stem cell of a mammal with a medium containing bone morphogenetic protein 4; and (b) culturing the cell after the step (a) with a medium containing a growth factor and a ROCK inhibitor.

EP 3 985 103 A1

SEQUENCE LISTING

<110> TOHOKU UNIVERSITY

<120> Cells having ability to differentiate to cells constituting placenta, and production method thereof

<130> PC-28011

<160> 26

<170> PatentIn version 3.5

<210> 1
<211> 1890
<212> DNA
<213> Homo sapiens

<220>
<221> CDS
<222> (211)..(1545)

<400> 1
gctaaacgac ccctccaaga taatttttaa aaaaccttct cctttgctca cctttgcttc 60

ccagccttcc catccccccca ccgaaagcaa atcattcaac gacccccgac cctccgacgg 120

caggagcccc ccgacctccc aggcggaccg ccctccctcc ccgcgcgcgg gttccgggcc 180

cggcgagagg gcgcgagcac agccgaggcc atg gag gtg acg gcg gac cag ccg 234
Met Glu Val Thr Ala Asp Gln Pro
1 5

cgc tgg gtg agc cac cac cac ccc gcc gtg ctc aac ggg cag cac ccg 282
Arg Trp Val Ser His His His Pro Ala Val Leu Asn Gly Gln His Pro
10 15 20

gac acg cac cac ccg ggc ctc agc cac tcc tac atg gac gcg gcg cag 330
Asp Thr His His Pro Gly Leu Ser His Ser Tyr Met Asp Ala Ala Gln
25 30 35 40

tac ccg ctg ccg gag gag gtg gat gtg ctt ttt aac atc gac ggt caa 378
Tyr Pro Leu Pro Glu Glu Val Asp Val Leu Phe Asn Ile Asp Gly Gln
45 50 55

ggc aac cac gtc ccg ccc tac tac gga aac tcg gtc agg gcc acg gtg 426
Gly Asn His Val Pro Pro Tyr Tyr Gly Asn Ser Val Arg Ala Thr Val
60 65 70

cag agg tac cct ccg acc cac cac ggg agc cag gtg tgc cgc ccg cct 474
Gln Arg Tyr Pro Pro Thr His His Gly Ser Gln Val Cys Arg Pro Pro
75 80 85

ctg ctt cat gga tcc cta ccc tgg ctg gac ggc ggc aaa gcc ctg ggc 522
Leu Leu His Gly Ser Leu Pro Trp Leu Asp Gly Gly Lys Ala Leu Gly
90 95 100

agc cac cac acc gcc tcc ccc tgg aat ctc agc ccc ttc tcc aag acg 570
Ser His His Thr Ala Ser Pro Trp Asn Leu Ser Pro Phe Ser Lys Thr
105 110 115 120

tcc atc cac cac ggc tcc ccg ggg ccc ctc tcc gtc tac ccc ccg gcc 618

```
Ser Ile His His Gly Ser Pro Gly Pro Leu Ser Val Tyr Pro Pro Ala
                125                 130                 135

tcg tcc tcc tcc ttg tcg ggg ggc cac gcc agc ccg cac ctc ttc acc        666
Ser Ser Ser Ser Leu Ser Gly Gly His Ala Ser Pro His Leu Phe Thr
            140                 145                 150

ttc ccg ccc acc ccg ccg aag gac gtc tcc ccg gac cca tcg ctg tcc        714
Phe Pro Pro Thr Pro Pro Lys Asp Val Ser Pro Asp Pro Ser Leu Ser
        155                 160                 165

acc cca ggc tcg gcc ggc tcg gcc cgg cag gac gag aaa gag tgc ctc        762
Thr Pro Gly Ser Ala Gly Ser Ala Arg Gln Asp Glu Lys Glu Cys Leu
    170                 175                 180

aag tac cag gtg ccc ctg ccc gac agc atg aag ctg gag tcg tcc cac        810
Lys Tyr Gln Val Pro Leu Pro Asp Ser Met Lys Leu Glu Ser Ser His
185                 190                 195                 200

tcc cgt ggc agc atg acc gcc ctg ggt gga gcc tcc tcg tcg acc cac        858
Ser Arg Gly Ser Met Thr Ala Leu Gly Gly Ala Ser Ser Ser Thr His
                205                 210                 215

cac ccc atc acc acc tac ccg ccc tac gtg ccc gag tac agc tcc gga        906
His Pro Ile Thr Thr Tyr Pro Pro Tyr Val Pro Glu Tyr Ser Ser Gly
            220                 225                 230

ctc ttc ccc ccc agc agc ctg ctg ggc ggc tcc ccc acc ggc ttc gga        954
Leu Phe Pro Pro Ser Ser Leu Leu Gly Gly Ser Pro Thr Gly Phe Gly
        235                 240                 245

tgc aag tcc agg ccc aag gcc cgg tcc agc aca gaa ggc agg gag tgt       1002
Cys Lys Ser Arg Pro Lys Ala Arg Ser Ser Thr Glu Gly Arg Glu Cys
    250                 255                 260

gtg aac tgt ggg gca acc tcg acc cca ctg tgg cgg cga gat ggc acg       1050
Val Asn Cys Gly Ala Thr Ser Thr Pro Leu Trp Arg Arg Asp Gly Thr
265                 270                 275                 280

gga cac tac ctg tgc aac gcc tgc ggg ctc tat cac aaa atg aac gga       1098
Gly His Tyr Leu Cys Asn Ala Cys Gly Leu Tyr His Lys Met Asn Gly
                285                 290                 295

cag aac cgg ccc ctc att aag ccc aag cga agg ctg tct gca gcc agg       1146
Gln Asn Arg Pro Leu Ile Lys Pro Lys Arg Arg Leu Ser Ala Ala Arg
            300                 305                 310

aga gca ggg acg tcc tgt gcg aac tgt cag acc acc aca acc aca ctc       1194
Arg Ala Gly Thr Ser Cys Ala Asn Cys Gln Thr Thr Thr Thr Thr Leu
        315                 320                 325

tgg agg agg aat gcc aat ggg gac cct gtc tgc aat gcc tgt ggg ctc       1242
Trp Arg Arg Asn Ala Asn Gly Asp Pro Val Cys Asn Ala Cys Gly Leu
    330                 335                 340

tac tac aag ctt cac aat att aac aga ccc ctg act atg aag aag gaa       1290
Tyr Tyr Lys Leu His Asn Ile Asn Arg Pro Leu Thr Met Lys Lys Glu
345                 350                 355                 360

ggc atc cag acc aga aac cga aaa atg tct agc aaa tcc aaa aag tgc       1338
Gly Ile Gln Thr Arg Asn Arg Lys Met Ser Ser Lys Ser Lys Lys Cys
                365                 370                 375
```

aaa aaa gtg cat gac tca ctg gag gac ttc ccc aag aac agc tcg ttt 1386
Lys Lys Val His Asp Ser Leu Glu Asp Phe Pro Lys Asn Ser Ser Phe
380 385 390

aac ccg gcc gcc ctc tcc aga cac atg tcc tcc ctg agc cac atc tcg 1434
Asn Pro Ala Ala Leu Ser Arg His Met Ser Ser Leu Ser His Ile Ser
395 400 405

ccc ttc agc cac tcc agc cac atg ctg acc acg ccc acg ccg atg cac 1482
Pro Phe Ser His Ser Ser His Met Leu Thr Thr Pro Thr Pro Met His
410 415 420

ccg cca tcc agc ctg tcc ttt gga cca cac cac ccc tcc agc atg gtc 1530
Pro Pro Ser Ser Leu Ser Phe Gly Pro His His Pro Ser Ser Met Val
425 430 435 440

acc gcc atg ggt tag agccctgctc gatgctcaca gggcccccag cgagagtccc 1585
Thr Ala Met Gly

tgcagtccct ttcgacttgc atttttgcag gagcagtatc atgaagccta aacgcgatgg 1645

atatatgttt ttgaaggcag aaagcaaaat tatgtttgcc actttgcaaa ggagctcact 1705

gtggtgtctg tgttccaacc actgaatctg gaccccatct gtgaataagc cattctgact 1765

catatcccct atttaacagg gtctctagtg ctgtgaaaaa aaaaatgctg aacattgcat 1825

ataacttata ttgtaagaaa tactgtacaa tgactttatt gcatctgggt agctgtaagg 1885

catga 1890

<210> 2
<211> 444
<212> PRT
<213> Homo sapiens

<400> 2

Met Glu Val Thr Ala Asp Gln Pro Arg Trp Val Ser His His His Pro
1 5 10 15

Ala Val Leu Asn Gly Gln His Pro Asp Thr His His Pro Gly Leu Ser
20 25 30

His Ser Tyr Met Asp Ala Ala Gln Tyr Pro Leu Pro Glu Glu Val Asp
35 40 45

Val Leu Phe Asn Ile Asp Gly Gln Gly Asn His Val Pro Pro Tyr Tyr
50 55 60

Gly Asn Ser Val Arg Ala Thr Val Gln Arg Tyr Pro Pro Thr His His
65 70 75 80

Gly Ser Gln Val Cys Arg Pro Pro Leu Leu His Gly Ser Leu Pro Trp
85 90 95

```
Leu Asp Gly Gly Lys Ala Leu Gly Ser His His Thr Ala Ser Pro Trp
            100                 105                 110

Asn Leu Ser Pro Phe Ser Lys Thr Ser Ile His His Gly Ser Pro Gly
        115                 120                 125

Pro Leu Ser Val Tyr Pro Pro Ala Ser Ser Ser Ser Leu Ser Gly Gly
    130                 135                 140

His Ala Ser Pro His Leu Phe Thr Phe Pro Pro Thr Pro Pro Lys Asp
145                 150                 155                 160

Val Ser Pro Asp Pro Ser Leu Ser Thr Pro Gly Ser Ala Gly Ser Ala
                165                 170                 175

Arg Gln Asp Glu Lys Glu Cys Leu Lys Tyr Gln Val Pro Leu Pro Asp
            180                 185                 190

Ser Met Lys Leu Glu Ser Ser His Ser Arg Gly Ser Met Thr Ala Leu
        195                 200                 205

Gly Gly Ala Ser Ser Ser Thr His His Pro Ile Thr Thr Tyr Pro Pro
    210                 215                 220

Tyr Val Pro Glu Tyr Ser Ser Gly Leu Phe Pro Pro Ser Ser Leu Leu
225                 230                 235                 240

Gly Gly Ser Pro Thr Gly Phe Gly Cys Lys Ser Arg Pro Lys Ala Arg
                245                 250                 255

Ser Ser Thr Glu Gly Arg Glu Cys Val Asn Cys Gly Ala Thr Ser Thr
            260                 265                 270

Pro Leu Trp Arg Arg Asp Gly Thr Gly His Tyr Leu Cys Asn Ala Cys
        275                 280                 285

Gly Leu Tyr His Lys Met Asn Gly Gln Asn Arg Pro Leu Ile Lys Pro
    290                 295                 300

Lys Arg Arg Leu Ser Ala Ala Arg Arg Ala Gly Thr Ser Cys Ala Asn
305                 310                 315                 320

Cys Gln Thr Thr Thr Thr Thr Leu Trp Arg Arg Asn Ala Asn Gly Asp
                325                 330                 335

Pro Val Cys Asn Ala Cys Gly Leu Tyr Tyr Lys Leu His Asn Ile Asn
            340                 345                 350
```

```
Arg Pro Leu Thr Met Lys Lys Glu Gly Ile Gln Thr Arg Asn Arg Lys
        355                 360                 365

Met Ser Ser Lys Ser Lys Lys Cys Lys Lys Val His Asp Ser Leu Glu
    370                 375                 380

Asp Phe Pro Lys Asn Ser Ser Phe Asn Pro Ala Ala Leu Ser Arg His
385                 390                 395                 400

Met Ser Ser Leu Ser His Ile Ser Pro Phe Ser His Ser Ser His Met
                405                 410                 415

Leu Thr Thr Pro Thr Pro Met His Pro Pro Ser Ser Leu Ser Phe Gly
            420                 425                 430

Pro His His Pro Ser Ser Met Val Thr Ala Met Gly
        435                 440


<210>  3
<211>  1568
<212>  DNA
<213>  Homo sapiens


<220>
<221>  CDS
<222>  (35)..(1477)

<400>  3
tctgcaccca gaccctgagc cgccgccgcc ggcc atg gag gtg gcg ccc gag cag       55
                                      Met Glu Val Ala Pro Glu Gln
                                      1               5

ccg cgc tgg atg gcg cac ccg gcc gtg ctg aat gcg cag cac ccc gac        103
Pro Arg Trp Met Ala His Pro Ala Val Leu Asn Ala Gln His Pro Asp
        10                  15                  20

tca cac cac ccg ggc ctg gcg cac aac tac atg gaa ccc gcg cag ctg        151
Ser His His Pro Gly Leu Ala His Asn Tyr Met Glu Pro Ala Gln Leu
    25                  30                  35

ctg cct cca gac gag gtg gac gtc ttc ttc aat cac ctc gac tcg cag        199
Leu Pro Pro Asp Glu Val Asp Val Phe Phe Asn His Leu Asp Ser Gln
40                  45                  50                  55

ggc aac ccc tac tat gcc aac ccc gct cac gcg cgg gcg cgc gtc tcc        247
Gly Asn Pro Tyr Tyr Ala Asn Pro Ala His Ala Arg Ala Arg Val Ser
                60                  65                  70

tac agc ccc gcg cac gcc cgc ctg acc gga ggc cag atg tgc cgc cca        295
Tyr Ser Pro Ala His Ala Arg Leu Thr Gly Gly Gln Met Cys Arg Pro
            75                  80                  85

cac ttg ttg cac agc ccg ggt ttg ccc tgg ctg gac ggg ggc aaa gca        343
His Leu Leu His Ser Pro Gly Leu Pro Trp Leu Asp Gly Gly Lys Ala
```

```
        90                  95                  100

gcc ctc tct gcc gct gcg gcc cac cac cac aac ccc tgg acc gtg agc        391
Ala Leu Ser Ala Ala Ala Ala His His His Asn Pro Trp Thr Val Ser
    105                 110                 115

ccc ttc tcc aag acg cca ctg cac ccc tca gct gct gga ggc cct gga        439
Pro Phe Ser Lys Thr Pro Leu His Pro Ser Ala Ala Gly Gly Pro Gly
120                 125                 130                 135

ggc cca ctc tct gtg tac cca ggg gct ggg ggt ggg agc ggg gga ggc        487
Gly Pro Leu Ser Val Tyr Pro Gly Ala Gly Gly Gly Ser Gly Gly Gly
                140                 145                 150

agc ggg agc tca gtg gcc tcc ctc acc cct aca gca gcc cac tct ggc        535
Ser Gly Ser Ser Val Ala Ser Leu Thr Pro Thr Ala Ala His Ser Gly
            155                 160                 165

tcc cac ctt ttc ggc ttc cca ccc acg cca ccc aaa gaa gtg tct cct        583
Ser His Leu Phe Gly Phe Pro Pro Thr Pro Pro Lys Glu Val Ser Pro
        170                 175                 180

gac cct agc acc acg ggg gct gcg tct cca gcc tca tct tcc gcg ggg        631
Asp Pro Ser Thr Thr Gly Ala Ala Ser Pro Ala Ser Ser Ser Ala Gly
    185                 190                 195

ggt agt gca gcc cga gga gag gac aag gac ggc gtc aag tac cag gtg        679
Gly Ser Ala Ala Arg Gly Glu Asp Lys Asp Gly Val Lys Tyr Gln Val
200                 205                 210                 215

tca ctg acg gag agc atg aag atg gaa agt ggc agt ccc ctg cgc cca        727
Ser Leu Thr Glu Ser Met Lys Met Glu Ser Gly Ser Pro Leu Arg Pro
                220                 225                 230

ggc cta gct act atg ggc acc cag cct gct aca cac cac ccc atc ccc        775
Gly Leu Ala Thr Met Gly Thr Gln Pro Ala Thr His His Pro Ile Pro
            235                 240                 245

acc tac ccc tcc tat gtg ccg gcg gct gcc cac gac tac agc agc gga        823
Thr Tyr Pro Ser Tyr Val Pro Ala Ala Ala His Asp Tyr Ser Ser Gly
        250                 255                 260

ctc ttc cac ccc gga ggc ttc ctg ggg gga ccg gcc tcc agc ttc acc        871
Leu Phe His Pro Gly Gly Phe Leu Gly Gly Pro Ala Ser Ser Phe Thr
    265                 270                 275

cct aag cag cgc agc aag gct cgt tcc tgt tca gaa ggc cgg gag tgt        919
Pro Lys Gln Arg Ser Lys Ala Arg Ser Cys Ser Glu Gly Arg Glu Cys
280                 285                 290                 295

gtc aac tgt ggg gcc aca gcc acc cct ctc tgg cgg cgg gac ggc acc        967
Val Asn Cys Gly Ala Thr Ala Thr Pro Leu Trp Arg Arg Asp Gly Thr
                300                 305                 310

ggc cac tac ctg tgc aat gcc tgt ggc ctc tac cac aag atg aat ggg       1015
Gly His Tyr Leu Cys Asn Ala Cys Gly Leu Tyr His Lys Met Asn Gly
            315                 320                 325

cag aac cga cca ctc atc aag ccc aag cga aga ctg tcg gcc gcc aga       1063
Gln Asn Arg Pro Leu Ile Lys Pro Lys Arg Arg Leu Ser Ala Ala Arg
        330                 335                 340

aga gcc ggc acc tgt tgt gca aat tgt cag acg aca acc acc acc tta       1111
```

```
Arg Ala Gly Thr Cys Cys Ala Asn Cys Gln Thr Thr Thr Thr Thr Leu
    345                 350                 355

tgg cgc cga aac gcc aac ggg gac cct gtc tgc aac gcc tgt ggc ctc       1159
Trp Arg Arg Asn Ala Asn Gly Asp Pro Val Cys Asn Ala Cys Gly Leu
360                 365                 370                 375

tac tac aag ctg cac aat gtt aac agg cca ctg acc atg aag aag gaa       1207
Tyr Tyr Lys Leu His Asn Val Asn Arg Pro Leu Thr Met Lys Lys Glu
                380                 385                 390

ggg atc cag act cgg aac cgg aag atg tcc aac aag tcc aag aag agc       1255
Gly Ile Gln Thr Arg Asn Arg Lys Met Ser Asn Lys Ser Lys Lys Ser
            395                 400                 405

aag aaa ggg gcg gag tgc ttc gag gag ctg tca aag tgc atg cag gag       1303
Lys Lys Gly Ala Glu Cys Phe Glu Glu Leu Ser Lys Cys Met Gln Glu
        410                 415                 420

aag tca tcc ccc ttc agt gca gct gcc ctg gct gga cac atg gca cct       1351
Lys Ser Ser Pro Phe Ser Ala Ala Ala Leu Ala Gly His Met Ala Pro
    425                 430                 435

gtg ggc cac ctc ccg ccc ttc agc cac tcc gga cac atc ctg ccc act       1399
Val Gly His Leu Pro Pro Phe Ser His Ser Gly His Ile Leu Pro Thr
440                 445                 450                 455

ccg acg ccc atc cac ccc tcc tcc agc ctc tcc ttc ggc cac ccc cac       1447
Pro Thr Pro Ile His Pro Ser Ser Ser Leu Ser Phe Gly His Pro His
                460                 465                 470

ccg tcc agc atg gtg acc gcc atg ggc tag ggaacagatg gacgtcgagg         1497
Pro Ser Ser Met Val Thr Ala Met Gly
            475                 480

accgggcact cccgggatgg gtggaccaaa cccttagcag cccagcattt cccgaaggcc     1557

gacaccactc c                                                          1568


<210>  4
<211>  480
<212>  PRT
<213>  Homo sapiens

<400>  4

Met Glu Val Ala Pro Glu Gln Pro Arg Trp Met Ala His Pro Ala Val
1               5                   10                  15


Leu Asn Ala Gln His Pro Asp Ser His His Pro Gly Leu Ala His Asn
            20                  25                  30


Tyr Met Glu Pro Ala Gln Leu Leu Pro Pro Asp Glu Val Asp Val Phe
        35                  40                  45


Phe Asn His Leu Asp Ser Gln Gly Asn Pro Tyr Tyr Ala Asn Pro Ala
    50                  55                  60
```

```
His Ala Arg Ala Arg Val Ser Tyr Ser Pro Ala His Ala Arg Leu Thr
65                  70                  75                  80

Gly Gly Gln Met Cys Arg Pro His Leu Leu His Ser Pro Gly Leu Pro
                85                  90                  95

Trp Leu Asp Gly Gly Lys Ala Ala Leu Ser Ala Ala Ala Ala His His
            100                 105                 110

His Asn Pro Trp Thr Val Ser Pro Phe Ser Lys Thr Pro Leu His Pro
        115                 120                 125

Ser Ala Ala Gly Gly Pro Gly Gly Pro Leu Ser Val Tyr Pro Gly Ala
    130                 135                 140

Gly Gly Gly Ser Gly Gly Gly Ser Gly Ser Ser Val Ala Ser Leu Thr
145                 150                 155                 160

Pro Thr Ala Ala His Ser Gly Ser His Leu Phe Gly Phe Pro Pro Thr
                165                 170                 175

Pro Pro Lys Glu Val Ser Pro Asp Pro Ser Thr Thr Gly Ala Ala Ser
            180                 185                 190

Pro Ala Ser Ser Ser Ala Gly Gly Ser Ala Ala Arg Gly Glu Asp Lys
        195                 200                 205

Asp Gly Val Lys Tyr Gln Val Ser Leu Thr Glu Ser Met Lys Met Glu
    210                 215                 220

Ser Gly Ser Pro Leu Arg Pro Gly Leu Ala Thr Met Gly Thr Gln Pro
225                 230                 235                 240

Ala Thr His His Pro Ile Pro Thr Tyr Pro Ser Tyr Val Pro Ala Ala
                245                 250                 255

Ala His Asp Tyr Ser Ser Gly Leu Phe His Pro Gly Gly Phe Leu Gly
            260                 265                 270

Gly Pro Ala Ser Ser Phe Thr Pro Lys Gln Arg Ser Lys Ala Arg Ser
        275                 280                 285

Cys Ser Glu Gly Arg Glu Cys Val Asn Cys Gly Ala Thr Ala Thr Pro
    290                 295                 300

Leu Trp Arg Arg Asp Gly Thr Gly His Tyr Leu Cys Asn Ala Cys Gly
305                 310                 315                 320
```

```
Leu Tyr His Lys Met Asn Gly Gln Asn Arg Pro Leu Ile Lys Pro Lys
                325                 330                 335

Arg Arg Leu Ser Ala Ala Arg Arg Ala Gly Thr Cys Cys Ala Asn Cys
            340                 345                 350

Gln Thr Thr Thr Thr Thr Leu Trp Arg Arg Asn Ala Asn Gly Asp Pro
        355                 360                 365

Val Cys Asn Ala Cys Gly Leu Tyr Tyr Lys Leu His Asn Val Asn Arg
    370                 375                 380

Pro Leu Thr Met Lys Lys Glu Gly Ile Gln Thr Arg Asn Arg Lys Met
385                 390                 395                 400

Ser Asn Lys Ser Lys Lys Ser Lys Lys Gly Ala Glu Cys Phe Glu Glu
                405                 410                 415

Leu Ser Lys Cys Met Gln Glu Lys Ser Ser Pro Phe Ser Ala Ala Ala
            420                 425                 430

Leu Ala Gly His Met Ala Pro Val Gly His Leu Pro Pro Phe Ser His
        435                 440                 445

Ser Gly His Ile Leu Pro Thr Pro Thr Pro Ile His Pro Ser Ser Ser
    450                 455                 460

Leu Ser Phe Gly His Pro His Pro Ser Ser Met Val Thr Ala Met Gly
465                 470                 475                 480


<210>  5
<211>  1420
<212>  DNA
<213>  Homo sapiens


<220>
<221>  CDS
<222>  (10)..(1311)

<400>  5
agagccgcg atg tcc ata ctt gcc aaa atg ggg gac tgg cag gac cgt cac       51
          Met Ser Ile Leu Ala Lys Met Gly Asp Trp Gln Asp Arg His
          1               5                   10

gac ggc acc agc aac ggg acg gca cgg ttg ccc cag ctg ggc act gta         99
Asp Gly Thr Ser Asn Gly Thr Ala Arg Leu Pro Gln Leu Gly Thr Val
15                  20                  25                  30

ggt caa tct ccc tac acg agc gcc ccg ccg ctg tcc cac acc ccc aat        147
Gly Gln Ser Pro Tyr Thr Ser Ala Pro Pro Leu Ser His Thr Pro Asn
                35                  40                  45
```

```
gcc gac ttc cag ccc cca tac ttc ccc cca ccc tac cag cct atc tac         195
Ala Asp Phe Gln Pro Pro Tyr Phe Pro Pro Pro Tyr Gln Pro Ile Tyr
            50                  55                  60

ccc cag tcg caa gat cct tac tcc cac gtc aac gac ccc tac agc ctg         243
Pro Gln Ser Gln Asp Pro Tyr Ser His Val Asn Asp Pro Tyr Ser Leu
        65                  70                  75

aac ccc ctg cac gcc cag ccg cag ccg cag cac cca ggc tgg ccc ggc         291
Asn Pro Leu His Ala Gln Pro Gln Pro Gln His Pro Gly Trp Pro Gly
    80                  85                  90

cag agg cag agc cag gag tct ggg ctc ctg cac acg cac cgg ggg ctg         339
Gln Arg Gln Ser Gln Glu Ser Gly Leu Leu His Thr His Arg Gly Leu
95                  100                 105                 110

cct cac cag ctg tcg ggc ctg gat cct cgc agg gac tac agg cgg cac         387
Pro His Gln Leu Ser Gly Leu Asp Pro Arg Arg Asp Tyr Arg Arg His
                115                 120                 125

gag gac ctc ctg cac ggc cca cac gcg ctc agc tca gga ctc gga gac         435
Glu Asp Leu Leu His Gly Pro His Ala Leu Ser Ser Gly Leu Gly Asp
            130                 135                 140

ctc tcg atc cac tcc tta cct cac gcc atc gag gag gtc ccg cat gta         483
Leu Ser Ile His Ser Leu Pro His Ala Ile Glu Glu Val Pro His Val
        145                 150                 155

gaa gac ccg ggt att aac atc cca gat caa act gta att aag aaa ggc         531
Glu Asp Pro Gly Ile Asn Ile Pro Asp Gln Thr Val Ile Lys Lys Gly
    160                 165                 170

ccc gtg tcc ctg tcc aag tcc aac agc aat gcc gtc tcc gcc atc cct         579
Pro Val Ser Leu Ser Lys Ser Asn Ser Asn Ala Val Ser Ala Ile Pro
175                 180                 185                 190

att aac aag gac aac ctc ttc ggc ggc gtg gtg aac ccc aac gaa gtc         627
Ile Asn Lys Asp Asn Leu Phe Gly Gly Val Val Asn Pro Asn Glu Val
                195                 200                 205

ttc tgt tca gtt ccg ggt cgc ctc tcg ctc ctc agc tcc acc tcg aag         675
Phe Cys Ser Val Pro Gly Arg Leu Ser Leu Leu Ser Ser Thr Ser Lys
            210                 215                 220

tac aag gtc acg gtg gcg gaa gtg cag cgg cgg ctc tca cca ccc gag         723
Tyr Lys Val Thr Val Ala Glu Val Gln Arg Arg Leu Ser Pro Pro Glu
        225                 230                 235

tgt ctc aac gcg tcg ctg ctg ggc gga gtg ctc cgg agg gcg aag tct         771
Cys Leu Asn Ala Ser Leu Leu Gly Gly Val Leu Arg Arg Ala Lys Ser
    240                 245                 250

aaa aat gga gga aga tct tta aga gaa aaa ctg gac aaa ata gga tta         819
Lys Asn Gly Gly Arg Ser Leu Arg Glu Lys Leu Asp Lys Ile Gly Leu
255                 260                 265                 270

aat ctg cct gca ggg aga cgt aaa gct gcc aac gtt acc ctg ctc aca         867
Asn Leu Pro Ala Gly Arg Arg Lys Ala Ala Asn Val Thr Leu Leu Thr
                275                 280                 285

tca cta gta gag gga gaa gct gtc cac cta gcc agg gac ttt ggg tac         915
Ser Leu Val Glu Gly Glu Ala Val His Leu Ala Arg Asp Phe Gly Tyr
```

290 295 300

gtg tgc gaa acc gaa ttt cct gcc aaa gca gta gct gaa ttt ctc aac 963
Val Cys Glu Thr Glu Phe Pro Ala Lys Ala Val Ala Glu Phe Leu Asn
305 310 315

cga caa cat tcc gat ccc aat gag caa gtg aca aga aaa aac atg ctc 1011
Arg Gln His Ser Asp Pro Asn Glu Gln Val Thr Arg Lys Asn Met Leu
320 325 330

ctg gct aca aaa cag ata tgc aaa gag ttc acc gac ctg ctg gct cag 1059
Leu Ala Thr Lys Gln Ile Cys Lys Glu Phe Thr Asp Leu Leu Ala Gln
335 340 345 350

gac cga tct ccc ctg ggg aac tca cgg ccc aac ccc atc ctg gag ccc 1107
Asp Arg Ser Pro Leu Gly Asn Ser Arg Pro Asn Pro Ile Leu Glu Pro
355 360 365

ggc atc cag agc tgc ttg acc cac ttc aac ctc atc tcc cac ggc ttc 1155
Gly Ile Gln Ser Cys Leu Thr His Phe Asn Leu Ile Ser His Gly Phe
370 375 380

ggc agc ccc gcg gtg tgt gcc gcg gtc acg gcc ctg cag aac tat ctc 1203
Gly Ser Pro Ala Val Cys Ala Ala Val Thr Ala Leu Gln Asn Tyr Leu
385 390 395

acc gag gcc ctc aag gcc atg gac aaa atg tac ctc agc aac aac ccc 1251
Thr Glu Ala Leu Lys Ala Met Asp Lys Met Tyr Leu Ser Asn Asn Pro
400 405 410

aac agc cac acg gac aac aac gcc aaa agc agt gac aaa gag gag aag 1299
Asn Ser His Thr Asp Asn Asn Ala Lys Ser Ser Asp Lys Glu Glu Lys
415 420 425 430

cac aga aag tga ggctctcctc ccgccccgcc cctcccacgc ctcaccagcc 1351
His Arg Lys

ccccgcgcgc ccaccctccg gcgggtgaca gctccgggat cagcaaccct tcctgctgct 1411

gctactgct 1420

<210> 6
<211> 433
<212> PRT
<213> Homo sapiens

<400> 6

Met Ser Ile Leu Ala Lys Met Gly Asp Trp Gln Asp Arg His Asp Gly
1 5 10 15

Thr Ser Asn Gly Thr Ala Arg Leu Pro Gln Leu Gly Thr Val Gly Gln
20 25 30

Ser Pro Tyr Thr Ser Ala Pro Pro Leu Ser His Thr Pro Asn Ala Asp
35 40 45

Phe Gln Pro Pro Tyr Phe Pro Pro Pro Tyr Gln Pro Ile Tyr Pro Gln

50 55 60

Ser Gln Asp Pro Tyr Ser His Val Asn Asp Pro Tyr Ser Leu Asn Pro
65 70 75 80

Leu His Ala Gln Pro Gln Pro Gln His Pro Gly Trp Pro Gly Gln Arg
85 90 95

Gln Ser Gln Glu Ser Gly Leu Leu His Thr His Arg Gly Leu Pro His
100 105 110

Gln Leu Ser Gly Leu Asp Pro Arg Arg Asp Tyr Arg Arg His Glu Asp
115 120 125

Leu Leu His Gly Pro His Ala Leu Ser Ser Gly Leu Gly Asp Leu Ser
130 135 140

Ile His Ser Leu Pro His Ala Ile Glu Glu Val Pro His Val Glu Asp
145 150 155 160

Pro Gly Ile Asn Ile Pro Asp Gln Thr Val Ile Lys Lys Gly Pro Val
165 170 175

Ser Leu Ser Lys Ser Asn Ser Asn Ala Val Ser Ala Ile Pro Ile Asn
180 185 190

Lys Asp Asn Leu Phe Gly Gly Val Val Asn Pro Asn Glu Val Phe Cys
195 200 205

Ser Val Pro Gly Arg Leu Ser Leu Leu Ser Ser Thr Ser Lys Tyr Lys
210 215 220

Val Thr Val Ala Glu Val Gln Arg Arg Leu Ser Pro Pro Glu Cys Leu
225 230 235 240

Asn Ala Ser Leu Leu Gly Gly Val Leu Arg Arg Ala Lys Ser Lys Asn
245 250 255

Gly Gly Arg Ser Leu Arg Glu Lys Leu Asp Lys Ile Gly Leu Asn Leu
260 265 270

Pro Ala Gly Arg Arg Lys Ala Ala Asn Val Thr Leu Leu Thr Ser Leu
275 280 285

Val Glu Gly Glu Ala Val His Leu Ala Arg Asp Phe Gly Tyr Val Cys
290 295 300

Glu Thr Glu Phe Pro Ala Lys Ala Val Ala Glu Phe Leu Asn Arg Gln
305 310 315 320

His Ser Asp Pro Asn Glu Gln Val Thr Arg Lys Asn Met Leu Leu Ala
325 330 335

Thr Lys Gln Ile Cys Lys Glu Phe Thr Asp Leu Leu Ala Gln Asp Arg
340 345 350

Ser Pro Leu Gly Asn Ser Arg Pro Asn Pro Ile Leu Glu Pro Gly Ile
355 360 365

Gln Ser Cys Leu Thr His Phe Asn Leu Ile Ser His Gly Phe Gly Ser
370 375 380

Pro Ala Val Cys Ala Ala Val Thr Ala Leu Gln Asn Tyr Leu Thr Glu
385 390 395 400

Ala Leu Lys Ala Met Asp Lys Met Tyr Leu Ser Asn Asn Pro Asn Ser
405 410 415

His Thr Asp Asn Asn Ala Lys Ser Ser Asp Lys Glu Glu Lys His Arg
420 425 430

Lys

<210> 7
<211> 22
<212> DNA
<213> Artificial sequence

<220>
<223> GATA3_F1 primer

<400> 7
gctaaacgac ccctccaaga ta 22

<210> 8
<211> 22
<212> DNA
<213> Artificial sequence

<220>
<223> GATA3_R\202P primer

<400> 8
tcatgcctta cagctaccca ga 22

<210> 9
<211> 18
<212> DNA

<213> Artificial sequence

<220>
<223> GATA2_F1 primer

<400> 9
tctgcaccca gaccctga 18

<210> 10
<211> 18
<212> DNA
<213> Artificial sequence

<220>
<223> GATA2_R1 primer

<400> 10
ggagtggtgt cggccttc 18

<210> 11
<211> 20
<212> DNA
<213> Artificial sequence

<220>
<223> TFAP2A_F1 primer

<400> 11
agagccgcga tgtccatact 20

<210> 12
<211> 21
<212> DNA
<213> Artificial sequence

<220>
<223> TFAP2A_R1 primer

<400> 12
agcagtagca gcagcaggaa g 21

<210> 13
<211> 20
<212> DNA
<213> Artificial sequence

<220>
<223> OCT4_F1 primer

<400> 13
gctcgagaag gatgtggtcc 20

<210> 14
<211> 20
<212> DNA
<213> Artificial sequence

<220>

<223> OCT4_R1 primer

<400> 14
cgttgtgcat agtcgctgct 20

<210> 15
<211> 20
<212> DNA
<213> Artificial sequence

<220>
<223> NANOG_F1 primer

<400> 15
gcagaaggcc tcagcaccta 20

<210> 16
<211> 19
<212> DNA
<213> Artificial sequence

<220>
<223> NANOG_R1 primer

<400> 16
aggttcccag tcgggttca 19

<210> 17
<211> 18
<212> DNA
<213> Artificial sequence

<220>
<223> GATA2_F2 primer

<400> 17
tcaagcccaa gcgaagac 18

<210> 18
<211> 18
<212> DNA
<213> Artificial sequence

<220>
<223> GATA2_R2 primer

<400> 18
cacaggcgtt gcagacag 18

<210> 19
<211> 20
<212> DNA
<213> Artificial sequence

<220>
<223> GATA3_F2 primer

<400> 19

ctcattaagc ccaagcgaag 20

<210> 20
<211> 20
<212> DNA
<213> Artificial sequence

<220>
<223> GATA3_R2 primer

<400> 20
tctgacagtt cgcacaggac 20

<210> 21
<211> 21
<212> DNA
<213> Artificial sequence

<220>
<223> TFAP2A_F2 primer

<400> 21
aacatgctcc tggctacaaa a 21

<210> 22
<211> 18
<212> DNA
<213> Artificial sequence

<220>
<223> TFAP2A_R2 primer

<400> 22
aggggagatc ggtcctga 18

<210> 23
<211> 22
<212> DNA
<213> Artificial sequence

<220>
<223> TP63_F1 primer

<400> 23
agaaacgaag atccccagat ga 22

<210> 24
<211> 22
<212> DNA
<213> Artificial sequence

<220>
<223> TP63_R1 primer

<400> 24
ctgttgctgt tgcctgtacg tt 22

```
<210>  25
<211>  30
<212>  DNA
<213>  Artificial sequence

<220>
<223>  ELF5_BSF1 primer

<400>  25
tgatggatat tgaatttgaa tttaaaggta                                        30


<210>  26
<211>  30
<212>  DNA
<213>  Artificial sequence

<220>
<223>  ELF5_BSR1 primer

<400>  26
caataaaaat aaaaacacct ataaccttat                                        30
```

## Description

### Technical Field

The present invention relates to cells capable of differentiating into placenta-forming cells, and to a method for producing same.

### Background Art

Trophoblasts are the main constituent of placental tissues. Trophoblast stem cells (TS cells) are considered highly beneficial for an understanding of the mechanism by which differentiation of trophoblast cells is regulated. To this end, there exists a need for establishment of a TS cell line that can be subcultured in a maintained undifferentiated state.

The present inventors have developed a method by which CD49f antibody positive cells collected from a cell suspension obtained from mammalian placental tissues are induced to differentiate into cells (TS-like cells) having similar characteristics to TS cells (PTL 1). However, the methods described in PTL 1 and NPL 1 require taking a cell suspension from placental tissues.

There are attempts to induce differentiation of ES cells into TS-like cells (NPL 2). However, the results of previous studies including analyses of gene expression pattern and DNA methylation pattern have revealed that many of these TS-like cells are not necessarily similar to TS cells.

### Citation List

### Patent Literature

PTL 1: Japanese Patent No. 6400832

### Non Patent Literature

NPL 1 : Okae H, et al., Derivation of Human Trophoblast Stem Cells. Cell Stem Cell. 2018 Jan; 22:50-63.
NPL 2: Gamage TK, et al., Stem cell insights into human trophoblast lineage differentiation. Hum Reprod Update. 2016 Dec; 23(1):77-103.

### Summary of Invention

### Technical Problem

As discussed above, previous attempts to induce differentiation of pluripotent stem cells into TS-like cells have been unsuccessful, and there is a need for development of a method that can induce differentiation of pluripotent stem cells into TS-like cells.

It is accordingly an object of the present invention to provide a method for producing TS-like cells from pluripotent stem cells. Another object is to provide TS-like cells produced by using the method.

### Solution to Problem

The present invention includes the following aspects.

[1] A cell derived from a pluripotent stem cell of a mammal and that is capable of differentiating into a placenta-forming cell, and that is negative for BRDT, and positive for TP63.
[2] The cell according to [1], which is negative for at least one selected from the group consisting of Oct4, Nanog, and Sox2.
[3] The cell according to [1] or [2], which is positive for at least one selected from the group consisting of GATA2, GATA3, and TFAP2A.
[4] The cell according to any one of [1] to [3], wherein a fraction of methylation of genomic DNA is 60% or less of whole genome.
[5] The cell according to any one of [1] to [4], wherein the placenta-forming cell is an extravillous cytotrophoblast or a syncytiotrophoblast.

[6] A method for producing a cell capable of differentiating into a placenta-forming cell,
the method including the steps of:

(a) culturing a pluripotent stem cell of a mammal with a medium containing bone morphogenetic protein 4 (BMP4); and
(b) culturing the cell after the step (a) with a medium containing a growth factor and a ROCK inhibitor.

[7] A method for producing a cell capable of differentiating into a placenta-forming cell,
the method including the steps of:

(a') introducing at least one gene selected from the group consisting of GATA2, GATA3, and TFAP2A to a pluripotent stem cell of a mammal; and
(b) culturing the cell after the step (a') with a medium containing a growth factor and a ROCK inhibitor.

[8] The method according to [6] or [7], wherein the medium in the step (b) further contains at least one selected from the group consisting of an ALK5 inhibitor and a GSK3β inhibitor.

Advantageous Effects of Invention

The present invention can provide a method for producing TS-like cells from pluripotent stem cells. The present invention can also provide TS-like cells produced by using the method.

Brief Description of Drawings

FIG. 1 shows a light micrograph of iPS cells, and light micrographs of TS-like cells derived from iPS cells.
FIG. 2 shows the result of a gene expression analysis of TS-like cells derived from iPS cells by introduction of GATA2, GATA3, or TFAP2A gene. The result is presented in a log2 (expression level) scale after calibrating the expression levels of TS cells and TS-like cells against the expression level of each gene in iPS cells taken as 1. The expression level of each gene in iPS cells is log2(1) = 0.
FIG. 3 shows immunostained images of TS-like cells (ES-TS) derived from ES cells treated with bone morphogenetic protein 4 (BMP4). Antigens against the antibodies used for immunostaining are shown on the left of the pictures.
FIG. 4A shows the result of a comparison of gene expression of TS-like cells (ES-TS; cells derived from ES cells treated with BMP4), TS cells, ES cells, and cells (ES-BMP4) prepared by conventional BMP4 treatment of ES cells.
FIG. 4B shows the result of a comparison of gene expression of TS-like cells (ES-TS; cells derived from ES cells treated with BMP4), TS cells, ES cells, and cells (ES-BMP4) prepared by conventional BMP4 treatment of ES cells.
FIG. 5 shows correlations of gene expression between TS-like cells (ES-TS; cells derived from ES cells treated with BMP4), TS cells, ES cells, and cells (ES-BMP4) prepared by conventional BMP4 treatment of ES cells.
FIG. 6 shows the result of an analysis of DNA methylation pattern of TS-like cells (ES-TS; cells derived from ES cells treated with BMP4), TS cells, ES cells, and cells (ES-BMP4) prepared by conventional BMP4 treatment of ES cells.
FIG. 7 shows the result of an analysis of DNA methylation pattern of ELF5 gene in TS-like cells (ES-TS; cells derived from ES cells treated with BMP4), TS cells, ES cells, and cells (ES-BMP4) prepared by conventional BMP4 treatment of ES cells.
FIG. 8A shows the result of immunostaining with anti-HLA-G antibodies performed for cells differentiated into extravillous cytotrophoblasts (EVTs) from TS-like cells (ES-TS) derived from ES cells treated with BMP4.
FIG. 8B shows the result of a differentiation test for differentiation of TS-like cells (ES-TS; cells derived from ES cells treated with BMP4) and TS cells into extravillous cytotrophoblasts (EVTs). The result is presented as levels of HLA-G expression compared for each cell type, with "ES-TS_EVT" representing cells differentiated into EVTs from TS-like cells, and "TS_EVT" representing cells differentiated into EVTs from TS cells.
FIG. 9A shows the result of immunostaining with anti-hCG antibodies performed for cells differentiated into syncytiotrophoblasts (STs) from TS-like cells (ES-TS) derived from ES cells treated with BMP4.
FIG. 9B shows the result of a differentiation test for differentiation of TS-like cells (ES-TS; cells derived from ES cells treated with BMP4) and TS cells into syncytiotrophoblasts (STs). The result is presented as levels of hCG expression compared for each cell type, with "ES-TS-ST" representing cells differentiated into STs from TS-like cells, and "TS_ST" representing cells differentiated into EVTs from TS cells.
FIG. 10 shows the result of a DNA methylation analysis for DNMT1 and ZFAT in TS-like cells (ES-TS; cells derived from ES cells treated with BMP4), TS cells, ES cells, and cells (ES-BMP4) prepared by conventional BMP4 treatment of ES cells.

FIG. 11 shows the result of a gene expression analysis for BRDT and SOHLH2 in TS-like cells (ES-TS; cells derived from ES cells treated with BMP4), TS cells, and ES cells.

Description of Embodiments

[Definitions]

As used herein, the terms "polynucleotide" and "nucleic acid" are interchangeable, and refer to a polymer of nucleotides linked by a phosphodiester bond. The polynucleotide and nucleic acid may be DNA or RNA, or a combination of DNA and RNA. The polynucleotide and nucleic acid may be a polymer of natural nucleotides, or a polymer of natural nucleotides and nonnatural nucleotides (nonnatural nucleotides are, for example, analogs of natural nucleotides, or nucleotides with a modification in at least one of the base, sugar, and phosphate moieties, such as a phosphorothioate backbone), or may be a polymer of nonnatural nucleotides.

In this specification, the sequences of bases in polynucleotides or nucleic acids are presented using the commonly accepted single-letter codes, unless otherwise specifically stated. Base sequences are presented from the 5' end to the 3' end, unless otherwise specifically stated.

In this specification, the nucleotide residues constituting polynucleotides or nucleic acids may be presented simply as adenine, thymine, cytosine, guanine, or uracil, or by using their customary single-letter codes.

As used herein, the term "gene" refers to a polynucleotide having at least one open reading frame encoding a specific protein. Genes may include both an exon and an intron.

As used herein, the terms "polypeptide", "peptide", and "protein" are interchangeable, and refer to polymers of amino acids joined by the amide bond. The polypeptide, peptide, or protein may be a polymer of natural amino acids, or a polymer of natural amino acids and nonnatural amino acids (nonnatural amino acids are, for example, chemical analogs or modified derivatives of natural amino acids), or may be a polymer of nonnatural amino acids. Amino acid sequences are presented from the N-terminus to the C-terminus, unless otherwise specifically stated.

As used herein, the phrase "to operably link " used in conjunction with polynucleotides means that a first base sequence is disposed close enough to a second base sequence, and that the first base sequence can exert its effects on the second base sequence or on a region placed under the control of the second base sequence. For example, by "to operably link a polynucleotide to a promoter", it means that a polynucleotide is linked to a promoter, and is expressible under the control of the promoter.

As used herein, the term "expressible state" refers to a state where a polynucleotide is transcribable in a cell to which the polynucleotide is introduced.

As used herein, the term "expression vector" refers to a vector containing a target polynucleotide and having a system by which the target polynucleotide is brought to an expressible state in a cell to which the vector is introduced.

[Cells (TS-Like Cells) Capable of Differentiating into Placenta-Forming Cells]

In an embodiment, there is provided a cell derived from a pluripotent stem cell of a mammal and that is capable of differentiating into a placenta-forming cell, and that is negative for BRDT, and positive for TP63.

The cell of the present embodiment is a TS-like cell having similar characteristics to TS cells. The TS-like cell has the capability to differentiate into placenta-forming cells, as does the TS cell. Examples of the placenta-forming cells include extravillous cytotrophoblasts (EVTs) and syncytiotrophoblasts (STs).

Whether cells have the potential to differentiate into EVTs can be determined by culturing cells under the conditions used to induce differentiation into EVTs, and checking whether the cells have differentiated into EVTs. Examples of conditions that can be used to induce differentiation into EVTs are described in the Examples section below. Specifically, cells can be induced to differentiate into EVTs as follows.

A collagen IV (Col IV)-coated plate containing EVT medium (see Examples) is inoculated with cells, and the cells are cultured after adding Matrigel in 2% of the medium volume. On day 3 after inoculation, the medium is changed to an EVT medium containing no NRG1, and the cells are further cultured after adding Matrigel in 0.5% of the medium volume. On day 6 after inoculation, the medium is changed to an EVT medium containing no NRG1 and KSR, and the cells are further cultured for 6 to 8 days after adding Matrigel in 0.5% of the medium volume.

Whether the cells have differentiated into EVTs after culture performed under these conditions to induce differentiation into EVTs can be confirmed by increased expression levels of HLA-G, aside from morphological observations using a microscope. HLA-G is an EVT marker, and its expression is upregulated by differentiation of TS cells into EVTs. It is accordingly possible to determine differentiation into EVTs when increased expression levels of HLA-G are observed in cultured cells as compared to uncultured cells under the foregoing conditions.

HLA-G (NCBI Gene ID: 3135) may be, for example, one registered with GenBank accession number NM_001363567.1 or NM_002127.5.

Whether cells have the potential to differentiate into STs can be determined by culturing cells under the conditions used to induce differentiation into STs, and checking whether the cells have differentiated into STs. Examples of conditions that can be used to induce differentiation into STs are described in the Examples section below. Specifically, cells can be induced to differentiate into STs as follows.

A collagen IV (Col IV)-coated plate containing ST medium (see Examples) is inoculated with cells, and the cells are cultured. On day 3 after inoculation, the medium is changed to a new ST medium, and the cells are further cultured for 3 days.

Whether the cells have differentiated into STs after culture performed under these conditions to induce differentiation into STs can be confirmed by increased expression levels of CGβ, aside from morphological observations using a microscope. CGβ (or CGB3 (chorionic gonadotropin subunit beta 3) as it is also called) is an ST marker, and its expression is upregulated by differentiation of TS cells into STs. It is accordingly possible to determine differentiation into STs when increased expression levels of CGβ are observed in cultured cells as compared to uncultured cells under the foregoing conditions.

Human CGβ (NCBI Gene ID: 1082) may be, for example, one registered with GenBank accession number NM-000737.3.

The cell of the present embodiment is a cell having similar characteristics to TS cells. However, the cell of the present embodiment is distinguishable from natural TS cells in that the cell of the present embodiment is BRDT negative. BRDT (bromodomain testis associated) is a protein with two bromodomain motifs and a PEST sequence (a cluster of proline, glutamic acid, serine, and threonine residues), characteristics of proteins that undergo rapid degradation. Human BRDT (NCBI Gene ID: 676) may be, for example, one registered with GenBank accession number NM_001242805.2, NM_001242806.2, NM_001242807.2, NM_001242808.2, NM_001242810.2, NM_001726.4, or NM_207189.3.

As used herein, the term "negative" used in conjunction with genes or proteins means that the proteins, or proteins encoded by the genes, are essentially undetectable. Here, "essentially undetectable" means that no detection can be made by ordinary means of protein detection. Such ordinary means of protein detection may be, for example, immunostaining using antibodies against the proteins.

Protein detection may be made by detecting mRNA, and a cell may be determined as being negative for a protein translated from the mRNA when the mRNA is essentially undetectable. For the measurement of mRNA, an ordinary method may be used, for example, such as a method using a next generation sequencer, and a quantitative real-time PCR method. For example, total mRNA extracted from a cell of interest may be analyzed with a next generation sequencer, and the cell can be determined as being negative for the target protein translated from the target mRNA when the target mRNA has a $\log_2$ (FPKM + 1) value of less than 1 (preferably less than 0.5, more preferably less than 0.1). FPKM (fragments per kilobase of exon per million reads mapped) is a value that can be obtained using a next generation sequencer, and the FPKM of a transcript t can be determined using the following formula.

$$FPKM = 10^9 \times X_t/(l_t N),$$

where Xt represents the number of short reads mapped for transcript t, $l_t$ represents the length (bp) of transcript t, and N represents the total number of short reads mapped.

As used herein, the term "positive" used in conjunction with genes or proteins means that the proteins, or proteins encoded by the genes, are essentially detectable. Here, "essentially detectable" means that detection can be made by ordinary means of protein detection (for example, immunostaining).

As above, a cell may be determined as being positive for a protein translated from corresponding mRNA when the mRNA is detectable by an ordinary method. For example, total mRNA extracted from a cell of interest may be analyzed with a next generation sequencer, and the cell can be determined as being negative for the target protein translated from the target mRNA when the target mRNA has a $\log_2$ (FPKM + 1) value of 1 or more (preferably 0.5 or more, more preferably 0.1 or more).

The cell of the present embodiment is a cell derived from a pluripotent stem cell. Here, "pluripotent stem cell" is a cell having pluripotency, and includes embryonic stem cells (ES cells), induced pluripotent stem cells (iPS cells), and pluripotent cells derived from these stem cells. The method of production of pluripotent stem cells is not particularly limited, and a known method may be used. ES or iPS cell lines are also available from cell banks, such as one in Riken BioResource Research Center (RIKEN BRC). Preferably, the pluripotent stem cells are ES cells or iPS cells.

The cell of the present embodiment is a TS-like cell derived by differentiation of a pluripotent stem cell using the method described below in the section [Method of Production of Cell (TS-like Cell) Capable of Differentiating into Placenta-Forming Cell]

The pluripotent stem cell is a mammalian pluripotent stem cell. The mammals are not particularly limited, and may be, for example, primates, rodents, or carnivores. Preferably, the mammals are primates. The primates are, for example, human, chimpanzee, rhesus, or marmoset, preferably human.

Aside from being capable of differentiating into placenta-forming cells, the cell of the present embodiment is positive for TP63, a characteristic similar to TS cells. In contrast, pluripotent stem cells, and TS-like cells induced by traditional methods are negative for TP63. That is, the cell of the present embodiment is similar to TS cells in that it is positive for TP63 as is TS cells, distinguishing itself from pluripotent stem cells and traditional TS-like cells.

Human TP63 (NCBI Gene ID: 8626) may be, for example, one registered with GenBank accession number NM_001114978.1, NM_001114979.1, NM_001114980.1, NM_001114981.1, NM_001114982.1, NM_001329144.1, NM_001329145.1, NM_001329146.1, NM_001329148.1, NM_001329149.1, NM_001329150.1, NM_001329964.1, or NM_003722.5.

Preferably, the cell of the present embodiment is negative for at least one selected from the group consisting of Oct4, Nanog, and Sox2. More preferably, the cell of the present embodiment is negative for at least two of Oct4, Nanog, and Sox2, even more preferably negative for all of Oct4, Nanog, and Sox2. These genes are known undifferentiation markers, and represent positive markers for pluripotent stem cells. Because the cell of the present embodiment is negative for these genes, the cell of the present embodiment is distinguishable from pluripotent stem cells from which the cell of the present embodiment is derived.

Human Oct4 (NCBI Gene ID: 5460) may be, for example, one registered with GenBank accession number NM_001173531.2, NM_001285986.1, NM_001285987.1, NM_002701.6, NM_203289.5, or Z11898.

Human Nanog (NCBI Gene ID: 79923) may be, for example, one registered with GenBank accession number NM_001297698.1, NM_024865.4, or AB093576.

Human Sox2 (NCBI Gene ID: 6657) may be, for example, one registered with GenBank accession number NM_003106.4.

Preferably, the cell of the present embodiment is positive for at least one selected from the group consisting of GATA2, GATA3, and TFAP2A. More preferably, the cell of the present embodiment is positive for at least two of GATA2, GATA3, and TFAP2A, even more preferably positive for all of GATA2, GATA3, and TFAP2A. These genes are TS cell markers. Specifically, being TP63 positive is an indication that the cell of the present embodiment has acquired the same functions possessed by natural TS cells, including the capability to differentiate into EVTs and STs, and that the cell of the present embodiment is distinguishable from pluripotent stem cells from which the cell of the present embodiment is derived.

Human GATA2 (NCBI Gene ID: 2624) may be, for example, one registered with GenBank accession number NM_001145661.1, NM_001145662.1, or NM_032638.4.

Human GATA3 (NCBI Gene ID: 2625) may be, for example, one registered with GenBank accession number NM_001002295.2 or NM_002051.2.

Human TFAP2A (NCBI Gene ID: 7020) may be, for example, one registered with GenBank accession number NM_001032280.2, NM_001042425.1, or NM_003220.3.

Preferably, the cell of the present embodiment is positive for genes such as TFAP2C, in addition to GATA2 and GATA3. TFAP2C is also a TS cell marker.

Human TFAP2C (NCBI Gene ID: 7022) may be, for example, one registered with GenBank accession number NM_003222.4.

Preferably, the cell of the present embodiment is negative for SOHLH2 (or $\log_2$ (FPKM + 1) < 1).

Human SOHLH2 (NCBI Gene ID: 54937) may be, for example, one registered with GenBank accession number NM_001282147.1 or NM_017826.3.

Preferably, the cell of the present embodiment has about the same fraction of genomic DNA methylation as TS cells. More specifically, the fraction of genomic DNA methylation is preferably 60% or less, more preferably 55% or less, even more preferably 50% or less, particularly preferably 47% or less of the whole genome.

In pluripotent stem cells, the fraction of genomic DNA methylation is about 81% of the whole genome. In TS cells, the fraction of genomic DNA methylation is about 45% of the whole genome. That is, the fraction of genomic DNA methylation can be used as an index of similarity to TS cells.

The lower limit fraction of genomic DNA methylation is, for example, preferably at least 35%, more preferably at least 40% of the whole genome. The preferred range of the fraction of genomic DNA methylation is, for example, 35% to 60%, more preferably 40% to 55%, even more preferably 40% to 47% of the whole genome.

The fraction of genomic DNA methylation in the cell can be measured by using a known method, for example, such as whole genome bisulfite sequencing (WGBS).

In the cell of the present embodiment, the fraction of DNA methylation in the promoter region (chrll: 34513753-34514270; transcription start site: chr11: 34513800)) of ELF5 gene is preferably 5% or less, more preferably 3% or less, even more preferably 2% or less.

In pluripotent stem cells, the fraction of DNA methylation in the promoter region of ELF5 gene is about 76%. In TS cells, the fraction of DNA methylation in the promoter region of ELF5 gene is about 1.7% of the whole genome.

That is, the fraction of DNA methylation in the promoter region of ELF5 gene can be used as an index of similarity to TS cells.

The lower limit fraction of DNA methylation in the promoter region of ELF5 gene is, for example, more than 0%, more preferably 0.5% or more, even more preferably 0.8% or more. The preferred range of the fraction of DNA methylation in the promoter region of ELF5 gene is, for example, more than 0% and 5% or less, more preferably 0.5% to 3%, even more preferably 0.8% to 2%.

Human ELF5 (NCBI Gene ID: 2001) may be, for example, one registered with GenBank accession number NM_001243080.1, NM_001243081.1, NM_001422.3, or NM_198381.1.

In the cell of the present embodiment, the fraction of DNA methylation in the promoter region (chr19: 10192830-10195739; transcription start site: chr19: 10195054)) of DNMT1 gene is preferably 20% or less, more preferably 15% or less, even more preferably 10% or less.

In natural TS cells, the fraction of DNA methylation in the promoter region of DNMT1 gene is about 40%. That is, the fraction of DNA methylation in the promoter region of DNMT1 gene can be used as an index that distinguishes natural TS cells from the cell of the present embodiment.

The lower limit fraction of DNA methylation in the promoter region of DNMT1 gene is, for example, 1% or more, more preferably 3% or more, even more preferably 5% or more. The preferred range of the fraction of DNA methylation in the promoter region of DNMT1 gene is, for example, 1% to 20%, more preferably 3% to 20%, even more preferably 5% to 15%, particularly preferably 5% to 10%.

Human DNMT1 (NCBI Gene ID: 1786) may be, for example, one registered with GenBank accession number NM_001130823.3, NM_001318730.1, NM_001318731.1, or NM_001379.3.

In the cell of the present embodiment, the fraction of DNA methylation in the promoter region (chr8: 134694984-134697871; transcription start site: chr8: 134696558)) of ZFAT gene is preferably 50% or more, more preferably 60% or more, even more preferably 70% or more, particularly preferably 80% or more.

In natural TS cells, the fraction of DNA methylation in the promoter region of ZFAT gene is about 40%. That is, the fraction of DNA methylation in the promoter region of ZFAT gene can be used as an index that distinguishes natural TS cells from the cell of the present embodiment.

The upper limit fraction of DNA methylation in the promoter region of ZFAT gene is, for example, 95% or less, more preferably 93% or less. The preferred range of the fraction of DNA methylation in the promoter region of ZFAT gene is, for example, 50% to 95%, more preferably 60% to 95%, even more preferably 70% to 95%, particularly preferably 80% to 93%.

Human ZFAT (NCBI Gene ID: 57623) may be, for example, one registered with GenBank accession number NM_001029939.3, NM_001167583.2, NM_001174157.1, NM_001174158.1, NM_001289394.1, or NM_020863.4.

The fractions of DNA methylation in the promoter regions of ELF5, DNMT1, and ZFAT genes can be measured by using a known method, for example, such as bisulfite sequencing or whole genome bisulfite sequencing (WGBS).

[Method of Production of Cell (TS-like Cell) Capable of Differentiating into Placenta-Forming Cell]

In an embodiment, the present invention provides a method for producing a cell capable of differentiating into a placenta-forming cell. The cell obtained by the producing method of the present embodiment is the TS-like cell of the embodiment above.

<First Embodiment>

The producing method of the present embodiment includes the steps of:

(a) culturing a pluripotent stem cell of a mammal with a medium containing bone morphogenetic protein 4; and
(b) culturing the cell after the step (a) with a medium containing a growth factor and a ROCK inhibitor.

Step (a)

Step (a) is a step of culturing a pluripotent stem cell of a mammal with a medium containing bone morphogenetic protein 4 (BMP4).

The mammalian pluripotent stem cell used in this step may be any of the cells exemplified in the foregoing section [Cells (TS-Like Cells) Capable of Differentiating into Placenta-Forming Cells]. The mammals are preferably primates, preferably human. The pluripotent stem cell is preferably an ES cell or an iPS cell.

The medium used in this step is a medium containing BMP4. The medium can be prepared by, for example, adding BMP4 to a base medium commonly used for animal cell culture. Examples of the base medium include Doulbecco's modified Eagle's medium (DMEM), DMEM/F12 medium, IMDM medium, Medium 199, Eagle's Minimum Essential medium (EMEM), αMEM medium, Ham's F12 medium, RPMI 1640 medium, Fischer's medium, and a mixed medium of these. The preferred base medium is, for example, DMEM/F12.

BMP4 may be any of various commercially available products.

The BMP4 concentration in the medium is not particularly limited, and may be, for example, 10 to 100 ng/mL, preferably 20 to 80 ng/mL, more preferably 30 to 70 ng/mL, even more preferably 40 to 60 ng/mL.

The base medium may be optionally supplemented with other components, in addition to BMP4. Examples of such other components include one or more serum replacements, for example, such as albumin, transferrin, sodium selenite, ITS-X (Invitrogen), a knockout serum replacement (KSR; an FBS serum replacement for ES cell culture), N2 supplement (Invitrogen), B27 supplement (Invitrogen), fatty acids, insulin, a collagen precursor, trace elements, 2-mercaptoethanol, and 3'-thiolglycerol. Other examples include one or more substances selected from a lipid, an amino acid, L-glutamine, GlutaMax, a non-essential amino acid, a vitamin, a growth factor, an antibiotic, an antioxidizing agent, pyruvic acid, a buffer, and a mineral salt. Preferred examples of the other components include KSR, GlutaMax, non-essential amino acids, 2-mercaptomethanol, and antibiotics (e.g., penicillin, streptomycin).

A specific example of the preferred media is the BMP4 medium described in the Examples section.

The culture temperature in this step may be a temperature commonly used for animal cell culture. The culture temperature may be, for example, 32 to 40°C, and may be appropriately selected in a temperature range of preferably 35 to 38°C.

The $CO_2$ concentration in a culture is not particularly limited, and is preferably about 2% to 5%, more preferably 5%.

The culture time in this step is not particularly limited, and may be, for example, 1 to 5 days (for example, 24 to 120 hours), preferably 2 to 4 days (for example, 48 to 96 hours), more preferably 3 days (for example, 50 to 80 hours).

In this step, it is preferable to start culture by inoculating a single-cell monolayer of pluripotent stem cells on a plate coated with Matrigel. Preferably, the medium is changed every 20 to 30 hours (for example, every 24 hours).

Step (b)

Step (b) is a step of culturing the cell after the step (a) with a medium containing a growth factor and a ROCK inhibitor.

The medium used in this step is a medium containing a growth factor and a ROCK inhibitor. The medium may be prepared by, for example, adding a growth factor and a ROCK inhibitor to a base medium commonly used for animal cell culture. The base medium may be any of the base media exemplified for step (a). The preferred base medium is, for example, DMEM/F12.

The growth factor is not particularly limited, and may be, for example, an epidermal growth factor (EGF), insulin, or a transforming growth factor (TGF). These may be commercially available growth factors. The growth factor is preferably an EGF.

The concentration of the growth factor in the medium is not particularly limited, and may be, for example, 10 to 100 ng/mL, preferably 20 to 80 ng/mL, more preferably 30 to 70 ng/mL, even more preferably 40 to 60 ng/mL.

The ROCK (rho associated coiled-coil containing protein kinase, or rho-associated kinase) inhibitor is not particularly limited, as long as it can inhibit the functions of rho-associated kinase. Examples of the ROCK inhibitor include trans-N-(4-pyridyl)-4-(1-aminoethyl)-cyclohexanecarboxamide, 1-(5-isoquinolinylsulfonyl)homopiperazine, and salts thereof. Other examples include low-molecular inhibitors such as Fasudil/HA1077, H-1152, and Wf-536, and derivatives thereof. Still other examples include antisense nucleic acids, siRNAs, and dominant negative mutants against ROCK, and expression vectors therefor.

Examples of commercially available products of trans-N-(4-pyridyl)-4-(1-aminoethyl)-cyclohexanecarboxamide or salts thereof include Y27632 ((R)-(+)-trans-N-(4-pyridyl)-4-(1-aminoethyl)-cyclohexanecarboxamide·2HCl·$H_2O$). The ROCK inhibitor may be used alone, or two or more ROCK inhibitors may be used in combination.

The ROCK inhibitor is preferably Y27632.

The concentration of the ROCK inhibitor in the medium is not particularly limited, and may be, for example, 0.1 to 50 μM, preferably 1 to 20 μM, more preferably 1 to 10 μM, even more preferably 3 to 8 μM.

The medium used in this step preferably contains at least one selected from the group consisting of an ALK5 inhibitor and a GSK3β inhibitor, in addition to a growth factor and a ROCK inhibitor.

The ALK5 inhibitor is not particularly limited, as long as it can inhibit ALK5 functions. Examples of the ALK5 inhibitor include 4-[4-(1,3-benzodioxol-5-yl)-5-(2-pyridyl)-lH-imidazol-2-yl]benzamide, or salts thereof. Other examples include low-molecular inhibitors such as A83-01 (3-(6-methylpyridin-2-yl)-N-phenyl-4-quinolin-4-ylpyrazole-1-carbothioamide), 2-(3-(6-methylpyridin-2-yl)-1H-pyrazol-4-yl)-1,5-naphthyridine, Wnt3a/BIO, GW788388, SM16, IN-1130, GW6604, SB-505124, and pyrimidine derivatives. Other examples include antisense nucleic acids, siRNAs, and dominant negative mutants against ALK5, and expression vectors therefor. Examples of commercially available products of 4-[4-(1,3-benzodioxol-5-yl)-5-(2-pyridyl)-1H-imidazol-2-yl]benzamide or salts thereof include SB431542. The ALK5 inhibitor may be used alone, or two or more ALK5 inhibitors may be used in combination.

Preferred for use as ALK5 inhibitor is SB431542 or A83-01, more preferably both SB431542 and A83-01.

The concentration of the ALK5 inhibitor in the medium is not particularly limited, and may be, for example, 0.1 to 20 μM, preferably 0.2 to 10 μM, more preferably 0.5 to 5 μM, even more preferably 0.5 to 3 μM. When SB431542 is used as ALK5 inhibitor, the SB431542 concentration in the medium may be, for example, 0.1 to 10 μM, preferably 0.2 to 5 μM, more preferably 0.5 to 3 μM, even more preferably 0.7 to 2 μM. When A83-01 is used as ALK5 inhibitor, the A83-01 concentration in the medium may be, for example, 0.1 to 5 μM, preferably 0.2 to 3 μM, more preferably 0.3 to 2 μM, even more preferably 0.3 to 1 μM.

The GSK3β inhibitor is not particularly limited, as long as it can inhibit GSK3β functions. Examples of the GSK3β inhibitor include 6-[[2-[[4-(2,4-dichlorophenyl)-5-(4-methyl-1H-imidazol-2-yl)-2-pyrimidinyl]amino]ethyl]amino]nicotinonitrile. Other examples include low-molecular inhibitors such as kenpaullone, 1-azakenpaullone, CHIR98014, AR-A014418, CT99021, CT20026, SB216763, AR-A014418, lithium, SB415286, TDZD-8, BIO, BIO-acetoxime, (5-methyl-1H-pyrazol-3-yl)-(2-phenylquinazolin-4-yl)amine, a pyridocarbazole-cyclopentadienyl ruthenium complex, TDZD-8 4-benzyl-2-methyl-1,2,4-thiadiazolidine-3,5-dione, 2-thio(3-iodobenzyl)-5-(1-pyridyl)-[1,3,4]-oxadiazole, OTDZT, alpha-4-dibromoacetophenone, AR-AO 144-18, 3-(1-(3-hydroxypropyl)-1H-pyrrolo[2,3-b]pyridin-3-yl]-4-pyrazin-2-yl-pyrrole-2,5-dione; TWSI 19 pyrrolopyrimidine compounds, L803 H-KEAPPAPPQSpP-NH2, or myristoylated forms of these; 2-chloro-1-(4,5-dibromo-thiophen-2-yl)-ethanone, SB216763, and SB415286. Still other examples include antisense nucleic acids, siRNAs, and dominant negative mutants against GSK3β, and expression vectors therefor. Examples of commercially available products of 6-[[2-[[4-(2,4-dichlorophenyl)-5-(4-methyl-1H-imidazol-2-yl)-2-pyrimidinyl]amino]ethyl]amino]nicotinonitrile include CHIR99021. The GSK3β inhibitor may be used alone, or two or more GSK3β inhibitors may be used in combination.

Preferred for use as GSK3β inhibitor is CHIR99021.

The concentration of the GSK3β inhibitor in the medium is not particularly limited, and may be, for example, 0.1 to 20 μM, preferably 0.2 to 10 μM, more preferably 0.5 to 5 μM, even more preferably 0.5 to 3 μM.

The medium used in this step may be supplemented with a histone deacetylase (HDAC) inhibitor, in addition to the foregoing components.

The HDAC inhibitor is not particularly limited, as long as it can inhibit HDAC functions. Examples of the HDAC inhibitor include low-molecular inhibitors such as valproic acid (VPA), trichostatin A, sodium butyrate, MC1293, and M344. Other examples include antisense nucleic acids, siRNAs, and dominant negative mutants against HDAC, and expression vectors therefor. The HDAC inhibitor may be used alone, or two or more HDAC inhibitors may be used in combination.

Preferred for use as HDAC inhibitor is VPA.

The concentration of the HDAC inhibitor in the medium is not particularly limited, and may be, for example, 0.01 to 10 mM, preferably 0.1 to 5 mM, more preferably 0.5 to 2 mM, even more preferably 0.5 to 1 mM.

The base medium may be optionally supplemented with other components, in addition to the foregoing components. Examples of such other components include sera (e.g., fetal bovine serum (FBS)), and one or more serum replacements, for example, such as albumin, transferrin, sodium selenite, ITS-X (Invitrogen), a knockout serum replacement (KSR; an FBS serum replacement for ES cell culture), N2 supplement (Invitrogen), B27 supplement (Invitrogen), fatty acids, insulin, a collagen precursor, trace elements, 2-mercaptoethanol, and 3'-thiolglycerol. Other examples include one or more substances selected from a lipid, an amino acid, L-glutamine, GlutaMax, a non-essential amino acid, a vitamin, a growth factor, an antibiotic, an antioxidizing agent, pyruvic acid, a buffer, and a mineral salt. Preferred examples of the other components include FBS, bovine serum albumin (BSA), ITS-X, L-ascorbic acid, 2-mercaptomethanol, and antibiotics (e.g., penicillin, streptomycin).

A specific example of the preferred medium is the TS medium described in the Examples section.

The culture temperature in this step may be a temperature commonly used for animal cell culture. The culture temperature may be, for example, 32 to 40°C, and may be appropriately set in a temperature range of preferably 35 to 38°C.

The $CO_2$ concentration in a culture is not particularly limited, and is preferably about 2% to 5%, more preferably 5%.

The culture time in this step is not particularly limited, and may be, for example, 7 days or longer, preferably 14 days or longer, more preferably 21 days or longer, even more preferably 28 days or longer. The upper limit of culture time is not particularly limited. The TS-like cell can be obtained by culturing the cultured cell after step (a) with the medium used in this step. The TS-like cell obtained can be maintained in an undifferentiated state by subculturing the as-cultured TS-like cell with the medium used in this step.

In this step, it is preferable to start culture by inoculating the cell after step (a) on a plate coated with collagen IV (Col IV). Preferably, the cell is subcultured every week (every 7 days). The cell after step (a) may be released from the culture plate with trypsin or a trypsin replacement (e.g., TrypLE), and may be inoculated to a culture plate containing the medium used in this step.

<Second Embodiment>

The producing method of the present embodiment includes the steps of:

(a') introducing at least one gene selected from the group consisting of GATA2, GATA3, and TFAP2A to a pluripotent stem cell of a mammal; and
(b) culturing the cell after the step (a') with a medium containing a growth factor and a ROCK inhibitor.

Step (a')

Step (a') is a step of introducing at least one gene selected from the group consisting of GATA2, GATA3, and TFAP2A to a pluripotent stem cell of a mammal.
The mammalian pluripotent stem cell used in this step may be any of the cells exemplified in the foregoing section [Cells (TS-Like Cells) Capable of Differentiating into Placenta-Forming Cells]. The mammals are preferably primates, preferably human. The pluripotent stem cell is preferably an ES cell or an iPS cell.
The gene introduced into a mammalian pluripotent stem cell is at least one gene selected from the group consisting of GATA2, GATA3, and TFAP2A. These genes may be introduced alone, or two or more of these genes may be introduced in combination. Preferably, any one of GATA2, GATA3, and TFAP2A is introduced.
The organism from which the GATA2, GATA3, and TFAP2A genes are derived is preferably the same organism from which the pluripotent stem cell is derived. For example, when using human pluripotent stem cells, it is preferable to use human GATA2, GATA3, and TFAP2A genes. The human GATA2, GATA3, and TFAP2A genes may have, for example, the base sequences presented in the foregoing section [Cells (TS-Like Cells) Capable of Differentiating into Placenta-Forming Cells]. Information of base sequences of corresponding genes from other mammals is available from known database, such as the GenBank sequence database.
The GATA2, GATA3, and TFAP2A genes are not limited to the wild-type genes, and may include a mutation (deletion, substitution, insertion, or addition), provided that the GATA2, GATA3, and TFAP2A genes with such mutations have inductive capability for TS-like cells. Here, "inductive capability for TS-like cells" means the function to induce differentiation of pluripotent stem cells into TS-like cells that come to have the properties described in the foregoing section [Cells (TS-Like Cells) Capable of Differentiating into Placenta-Forming Cells] after step (b) following introduction of the genes into pluripotent stem cells.
Examples of GATA2, GATA3, or TFAP2A gene that can be used in this step include the genes listed in (A) to (G) below.

(A) Wild-type GATA2, GATA3, or TFAP2A gene (e.g., a polynucleotide consisting of the nucleotide sequence represented by SEQ ID NO: 3 (GATA2), SEQ ID NO: 1 (GATA3), or SEQ ID NO: 5 (TFAP2A).
(B) A polynucleotide consisting of a nucleotide sequence that codes for wild-type GATA2, GATA3, or TFAP2A protein (e.g., a protein consisting of the amino acid sequence represented by SEQ ID NO: 4 (GATA2), SEQ ID NO: 2 (GATA3), or SEQ ID NO: 6 (TFAP2A)).
(C) A polynucleotide that codes for a protein consisting of the amino acid sequence of wild-type GATA2, GATA3, or TFAP2A protein (e.g., the amino acid sequence represented by SEQ ID NO: 4 (GATA2), SEQ ID NO: 2 (GATA3), or SEQ ID NO: 6 (TFAP2A)) with mutation of one or a plurality of amino acids, and that has inductive capability for TS-like cells.
(D) A polynucleotide that codes for a protein consisting of an amino acid sequence with at least 70% sequence identity with the amino acid sequence of wild-type GATA2, GATA3, or TFAP2A protein (e.g., the amino acid sequence represented by SEQ ID NO: 4 (GATA2), SEQ ID NO: 2 (GATA3), or SEQ ID NO: 6 (TFAP2A)), and that has inductive capability for TS-like cells.
(E) A polynucleotide consisting of a nucleotide sequence with mutation in one or a plurality of the nucleotides of wild-type GATA2, GATA3, or TFAP2A gene (e.g., a polynucleotide consisting of the nucleotide sequence represented by SEQ ID NO: 3 (GATA2), SEQ ID NO: 1 (GATA3), or SEQ ID NO: 5 (TFAP2A)), and that has inductive capability for TS-like cells.
(F) A polynucleotide consisting of a nucleotide sequence with at least 70% sequence identity with wild-type GATA2, GATA3, or TFAP2A gene (e.g., a polynucleotide consisting of the nucleotide sequence represented by SEQ ID NO: 3 (GATA2), SEQ ID NO: 1 (GATA3), or SEQ ID NO: 5 (TFAP2A)), and that has inductive capability for TS-like cells.
(G) A polynucleotide that hybridizes under stringent conditions with wild-type GATA2, GATA3, or TFAP2A gene (e.g., a polynucleotide consisting of the nucleotide sequence represented by SEQ ID NO: 3 (GATA2), SEQ ID NO: 1 (GATA3), or SEQ ID NO: 5 (TFAP2A)), and that has inductive capability for TS-like cells.

In (C) and (E) above, "mutation" may be any of deletion, substitution, addition, and insertion, or a combination of these.

In (C) above, the number of "plurality" is not particularly limited, and may be, for example, 2 to 30, preferably 2 to 20, more preferably 2 to 10, even more preferably 2 to 5, particularly preferably 2 or 3.

In (E) above, the number of "plurality" is not particularly limited, and may be, for example, 2 to 60, preferably 2 to 50, more preferably 2 to 40 or 2 to 30, even more preferably 2 to 20 or 2 to 10, particularly preferably 2 to 5, or 2 or 3.

In (D) and (F) above, the sequence identity is not particularly limited, as long as it is at least 70%. However, the sequence identity is preferably at least 80%, more preferably at least 85%, even more preferably at least 95%. The sequence identity between amino acid sequences or between nucleotide sequences is determined as a percentage of matching amino acids or matching nucleotides relative to the whole amino acid sequence or nucleotide sequence, excluding gaps in the alignment, when two amino acid sequences or nucleotide sequences are paired to create the greatest number of matches for corresponding amino acids or corresponding nucleotides with gaps interposed in positions equivalent of insertions and deletions. The sequence identity between amino acid sequences or between nucleotide sequences can be determined using various types of homology search software known in the art. For example, the sequence identity between amino acid sequences or between nucleotide sequences can be obtained by calculations based on an alignment obtained by the known homology search software BLASTP.

In (G) above, the stringent conditions are, for example, conditions described in Molecular Cloning - A LABORATORY MANUAL THIRD EDITION (Sambrook et al., Cold Spring Harbor Laboratory Press). As an example, polynucleotides are hybridized by being incubated for several hours to overnight at 42 to 70°C in a hybridization buffer consisting of 6 × SSC (a 20 × SSC composition: a 3 M sodium chloride and 0.3 M citric acid solution, pH 7.0), a 5 × Denhardt's solution (100 × Denhardt's solution composition: 2 mass% bovine serum albumin, 2 mass% Ficoll, 2 mass% polyvinylpyrrolidone), 0.5 mass% SDS, 0.1 mg/mL salmon sperm DNA, and 50% formamide. The wash buffer used for washing after incubation is, for example, preferably a 0.1 mass% SDS-containing 1 × SSC solution, more preferably a 0.1 mass% SDS-containing 0.1 × SSC solution.

In (B) to (E) above, the degenerate codons are preferably codons with high frequencies of usage in pluripotent stem cells of the mammal used. For example, for human pluripotent stem cells, it is preferable to use codons with high frequencies of usage in human cells. That is, it is preferable to optimize codons for human.

The method used to introduce the genes into a pluripotent stem cell is not particularly limited, and a known method may be used. For example, the genes may be introduced into the target pluripotent stem cell by being cloned into an expression vector that can be expressed in the target pluripotent stem cell.

In addition to the genes, the expression vector preferably includes a promoter that regulates expression of the genes. In such an expression vector, the promoter is disposed upstream of the genes to regulate expression of the genes, and the genes are operably linked to the promoter.

Examples of the promoter include the SRα promoter, the SV40 early promoter, a LTR of a retrovirus, the CMV (cytomegalovirus) promoter, the RSV (Rous sarcomavirus) promoter, the HSV-TK (herps simplex virus thymidine kinase) promoter, the EF1α promoter, a metallothionein promoter, and a heat shock promoter. The promoter may be used with an enhancer for the IE gene of human CMV. As an example, it is possible to use the CAG promoter (containing a cytomegalovirus enhancer, a chicken β-actin promoter, and a polyA signal site of β-globin gene).

In addition to promoters, the expression vector may contain, for example, an enhancer, a polyA addition signal, a marker gene, a replication initiation site, and a gene that codes for a protein that regulates replication by binding to the replication initiation site, as desired. The marker gene refers to a gene that enables screening or selection of cells by being introduced into cells. Specific examples of the marker gene include a drug resistance gene, a fluorescence protein gene, a luminescent enzyme gene, and a chromogenic enzyme gene. These may be used alone, or two or more of these may be used in combination. Specific examples of the drug resistance gene include a neomycin resistance gene, a tetracycline resistance gene, a kanamycin resistance gene, a zeocin resistance gene, a hygromycin resistance gene, and a puromycin resistance gene. Specific examples of the fluorescence protein gene include a green fluorescence protein (GFP) gene, a yellow fluorescence protein (YFP) gene, and a red fluorescence protein (RFP) gene. Specific examples of the luminescent enzyme gene include a luciferase gene. Specific examples of the chromogenic enzyme gene include a β-galactosidase gene, a β-glucuronidase gene, and an alkali phosphatase gene.

The type of expression vector is not particularly limited, and known expression vectors may be used. Examples of expression vectors include episomal vectors, artificial chromosome vectors, plasmid vectors, and virus vectors.

Examples of the episomal vectors include vectors in which a sequence necessary for autonomous replication, such as those originating in EBV and SV40, is contained as a vector element. Specific examples of such vector elements necessary for autonomous replication include a replication initiation site, and a gene that codes for a protein that regulates replication by binding to the replication initiation site. Examples are replication initiation site oriP and EBNA-1 gene in the case of EBV, and replication initiation site ori and SV40LT gene in the case of SV40.

Examples of the artificial chromosome vectors include a YAC (yeast artificial chromosome) vector, a BAC (bacterial artificial chromosome) vector, and a PAC (P1-derived artificial chromosome) vector.

The plasmid vectors are not particularly limited, as long as the vectors can be expressed in a pluripotent stem cell to which the vectors are introduced. Examples of plasmid vectors for expression in animal cells include pA1-11, pXT1, pRc/CMV, pRc/RSV, and pcDNAI/Neo.

Examples of the virus vectors include retrovirus vectors (including lentivirus vectors), adenovirus vectors, adeno-associated virus vectors, Sendai virus vectors, herpes virus vectors, vaccinia virus vectors, poxvirus vectors, poliovirus vectors, sylbisvirus vectors, rhabdovirus vectors, paramyxovirus vectors, and orthomyxovirus vectors.

The method used to introduce an expression vector into a pluripotent stem cell is not particularly limited, and may be appropriately selected according to the type of expression vector. Examples of methods that can be used to introduce an expression vector into a pluripotent stem cell include the lipofection method, the microinjection method, the DEAE dextran method, the gene-gun technique, the electroporation method, and the calcium phosphate method. When the expression vector is a virus vector, for example, the polybrene technique may be used for infection of a pluripotent stem cell with the virus vector.

When the expression vector containing the genes is carrying an antibiotic resistance gene as a selectable marker, cells after the introduction of the expression vector can be efficiently selected by culturing the cells with a medium containing an antibiotic corresponding to the antibiotic resistance gene after the expression vector is introduced.

Step (b)

Step (b) is as described in step (b) of First Embodiment.

The producing method of the present embodiment enables a pluripotent stem cell to be induced to differentiate into a TS-like cell having the capability to differentiate into a placenta-forming cell. The TS-like cell obtain by the producing method of the present embodiment can be used as a research material for basic research, such as in studies of early development of mammals, and functional analyses of the placenta. The TS-like cell also can be used for an understanding of the pathogenesis of disorders associated with abnormal placenta, or for the development of therapeutic methods for such disorders. Other applicable areas include reproductive medicine and regenerative medicine.

Examples

The following describes the present invention through Examples. It is to be noted, however, that the present invention is not limited to the descriptions of the following Examples.

[Materials and Methods]

(TS Cell Culture)

A TS cell line was cultured following the previously described method (Okae, H., Toh, H., Sato, T. et al. Cell Stem Cell 22, 50-63 e56, doi:10.1016/j.stem.2017.11.004 (2018)). The TS cell line was cultured in a TS medium containing 5 μg/mL Col IV (354233; Corning, Corning, NY). The cells were cultured in 5% $CO_2$ at 37°C, and the culture medium was changed after 2 days.

The TS medium was prepared by adding the following components to DMEM/F12 (048-29785; Fujifilm Wako, Osaka, Japan).

0.1 mM 2-Mercaptoethanol (21985023; Thermo Fisher Scientific, Waltham, MA)
0.2% FBS (16141-079; Thermo Fisher Scientific)
0.5% Penicillin-Streptomycin (15140122; Thermo Fisher Scientific)
0.3% BSA (017-22231; Fujifilm Wako)
1% ITS-X supplement (094-06761; Fujifilm Wako)
1.5 μg/ml L-Ascorbic acid (013-12061; Fujifilm Wako)
50 ng/mL EGF (053-07871; Fujifilm Wako)
2 μM CHIR99021 (038-23101; Fujifilm Wako)
0.5 μM A83-01 (035-24113; Fujifilm Wako)
1 μM SB431542 (031-24291; Fujifilm Wako)
0.8 mM VPA (227-01071; Fujifilm Wako)
5 μM Y-27632 (036-24023; Fujifilm Wako)

(Culture of ES cells and iPS cells)

A human ES cell line (SEES 1, 4, 6) and an iPS cell line (Nips-B2) were cultured on a Matrigel (354234; Corning) in an ES medium [hPSC medium delta (197-17571; Fujifilm Wako) supplemented with 35 ng/ml FGF2 (064-05381;

Fujifilm Wako) and 10 μM Y-27632 (Fujifilm Wako)], following the manufacturer's protocol. The cells were subcultured after dissociating the colonies with TrypLE expression enzyme (12604021; Thermo Fisher Scientific) by allowing the enzyme to act at 37°C for 5 minutes. The cells were cultured in 5% $CO_2$ at 37°C. On the following day, the medium was changed to an ES medium containing no Y-27632, and, subsequently, this medium was used daily as a new medium.

(Induced Differentiation into TS-Like Cells via BMP4 Signaling)

Single monolayer ES cells or iPS cells were inoculated on a Matrigel-coated plate containing ES medium (10,000 cells/$cm^2$), and cultured in 5% $CO_2$ at 37°C. On the following day, the medium was changed to a BMP4 medium (Krendl, C., Shaposhnikov, D., Rishko, V. et al. Proc Natl Acad Sci USA 114, E9579-E9588, doi:10.1073/pnas.1708341114 (2017)). A fresh BMP4 medium was used every 24 hours. On day 3, the cells were dissociated with TrypLE (Thermo Fisher Scientific) by allowing the enzyme to act at 37°C for 13 to 15 minutes. At a split ratio of 1:2, the cells were inoculated on a Col IV-coated plate containing a TS medium, and cultured in 5% $CO_2$ at 37°C before subculturing the cells every week. The ES cells or iPS cells after BMP4 treatment were a heterogeneous population after the first passage. However, TS-like colonies, capable of self replication, appeared after 4 passages. Thereafter, the ES cells or iPS cells after BMP4 treatment were regularly subcultured every week at a 1:5 to 1:10 split ratio.

The BMP4 medium was prepared by adding the following components to DMEM/F12 (048-29785; Fujifilm Wako, Osaka, Japan).

20% Knockout serum replacement (1082802 8; Thermo Fisher Scientific)
1% GlutaMax (35050038; Thermo Fisher Scientific)
1% Nonessential amino acids (1140050; Thermo Fisher Scientific)
0.1 mM 2-Mercaptoethanol (21985023; Thermo Fisher Scientific)
1% Penicillin-Streptomycin (Thermo Fisher Scientific)
50 ng/mL BMP4 (020-18851; Fujifilm Wako)

(Induced Differentiation into TS-Like Cells by Gene Introduction)

cDNA was cloned into a pLVSIN-EF1α base transfer vector, using an In-Fusion® HD cloning kit (Takara Bio, Shiga, Japan). After codon optimization, cDNA of GATA2, GATA3, or TFAP2A (GATA2: SEQ ID NO: 1, 2; GATA3: SEQ ID NO: 3, 4; TFAP2A: SEQ ID NO: 5, 6) was synthesized in pLVSIN-EF1α vector, and transferred to a lentivector selectable by puromycin. Single monolayer iPS cells were inoculated on a Matrigel-coated plate containing an ES medium (100,000 cells/$cm^2$). The medium was then changed to a lentivirus stock supplemented with 6 μg/mL polybrene (Sigma-Aldrich, St-Louis, MO). After 24 hours from infection, the cells were cultured for 2 days in 0.5 to 1.0 μg/mL puromycin for selection. After selection, the iPS cells were inoculated at a 1:10 split ratio to a Col IV-coated plate containing a TS medium, and cultured in 5% $CO_2$ at 37°C before subculturing the cells every week. iPS cells that incorporated the gene were a heterogeneous population after the first passage. However, TS-like colonies, capable of self replication, appeared after 4 passages. Thereafter, the iPS cells that had incorporated the gene were regularly subcultured every week at a 1:5 to 1:10 split ratio.

The primers (5'-3') used for cloning of GATA2, GATA3, and TFAP2A are as follows. Here and below, "F" means a forward primer, and "R" a reverse primer.

GATA3_F: GCTAAACGACCCCTCCAAGATA (SEQ ID NO: 7)
GATA3_R: TCATGCCTTACAGCTACCCAGA (SEQ ID NO: 8)
GATA2_F: TCTGCACCCAGACCCTGA (SEQ ID NO: 9)
GATA2_R: GGAGTGGTGTCGGCCTTC (SEQ ID NO: 10)
TFAP2A_F: AGAGCCGCGATGTCCATACT (SEQ ID NO: 11)
TFAP2A_R: AGCAGTAGCAGCAGCAGGAAG (SEQ ID NO: 12)

(Differentiation into EVTs or STs)

Methods for induced differentiation into extravillous cytotrophoblasts (EVTs) and syncytiotrophoblasts (STs) are previously described (Okae, H., Toh, H., Sato, T. et al. Cell Stem Cell 22, 50-63 e56, doi:10.1016/j.stem.2017.11.004 (2018)). In order to induce TS cells or TS-like cells to differentiate into EVT cells, TS cells and TS-like cells were inoculated on a 1 μg/mL Col IV-coated plate (Corning) containing an EVT medium, at a density of 8,000 cells/$cm^2$. After the inoculation of the cells to the medium, a Matrigel (Corning) was added in 2% of the medium volume. On day 3 after inoculation, the medium was changed to an EVT medium containing no NRG1 (Cell Signaling), and a Matrigel (Corning) was added in 0.5% of the medium volume. On day 6 after inoculation, the medium was changed to an EVT medium containing no NRG1 (Cell Signaling) and KSR (Thermo Fisher Scientific), and a Matrigel (Corning) was added in 0.5% of the medium volume. The cells were analyzed after 6 to 8 days from the last medium change.

The EVT medium was prepared by adding the following components to DMEM/F12 (048-29785; Fujifilm Wako, Osaka, Japan).

0.5% Penicillin-Streptomycin (Thermo Fisher Scientific)
0.3% BSA (Fujifilm Wako)
1% ITS-X supplement (Fujifilm Wako)
100 ng/ml NRG1 (5218SC; Cell Signaling)
7.5 μM A83-01 (Fujifilm Wako)
2.5 μM Y27632 (Fujifilm Wako)
4% KSR (Thermo Fisher Scientific)

In order to induce differentiation of TS cells or TS-like cells to ST cells, TS cells and TS-like cells were inoculated on a 2.5 μg/mL Col IV-coated plate (Corning) containing an ST medium, at a density of 10,000 cells/$cm^2$. The medium was changed on day 3 after inoculation, and the cells were analyzed on day 6 after inoculation.

The ST medium was prepared by adding the following components to DMEM/F12 (048-29785; Fujifilm Wako, Osaka, Japan).

0.1 mM 2-Mercaptoethanol (Thermo Fisher Scientific)
0.5% Penicillin-Streptomycin (Thermo Fisher Scientific)
0.3% BSA (Fujifilm Wako)
1% ITS-X supplement (Fujifilm Wako)
2.5 μM Y27632 (Fujifilm Wako)
2 μM Forskolin (Fujifilm Wako)
4% KSR (Thermo Fisher Scientific)

(RNA Sequencing)

Total RNA was extracted with an RNeasy® Mini Kit and an RNase-Free DNase (QIAGEN, Valencia, CA), and was used for library construction with a TruSeq® Stranded mRNA LT Sample Prep Kit (Illumina, San Diego, CA) following the manufacturer's protocol. The integrity of RNA was evaluated using a TapeStation 2200 (Agilent Technologies, Santa Clara, CA). All samples had RINe values of more than 9. The library was sequenced with 101 bp paired-end reads using an Illumina HiSeq 2500 Platform (Illumina). Reads were aligned against reference genome (UCSC hg38) using a TopHat with a Refseq gene annotation (Trapnell, C., Roberts, A., Goff, L. et al. Nat Protoc 7, 562-578, doi:10.1038/nprot.2012.016 (2012)). For female samples, the Y chromosome was excluded from reference genome. The expression levels (FPKM) of Refseq gene were calculated with Cufflinks (Trapnell, C., Roberts, A., Goff, L. et al. Nat Protoc 7, 562-578, doi:10.1038/nprot.2012.016 (2012)). X-linked genes and Y-linked genes in the pseudoautosomal region were excluded from analysis.

(Whole-Genome Bisulfite Sequencing (WGBS))

WGBS was performed by post-bisulfite adaptor tagging (PBAT) (Miura, F., Enomoto, Y., Dairiki, R. et al. Nucleic Acids Res 40, e136, doi:10.1093/nar/gks454 (2012)). In brief, genomic DNA was purified by phenol/chloroform extraction and ethanol precipitation. Genomic DNA with 0.5% (w/w) unmethylated λ phage DNA (Promega, Madison, WI) was used to prepare a library, following the PBAT protocol. The concentration of the PBAT product was quantified with a KAPA Library Quantification Kit for Illumina platforms (KAPA Biosystems, Woburn, MA). The PBAT library was sequenced with 101 bp single-end reads using an Illumina HiSeq 1500 equipped with HCS v2.0.5 and RTA v1.17.20 (Illumina). Reads were aligned against reference genome using a Bismark (Krueger, F. & Andrews, S. R. Bismark: Bioinformatics 27, 1571-1572, doi:10.1093/bioinformatics/btrl67 (2011)). The methylation level (UCSC hg38) of each cytosine was calculated using a Bismark Methylation Extractor. For calculations of methylation level at each CpG site, reads from both strands were combined. The methylation level of CpG covered by at least 5 reads was analyzed.

(Quantitative Real-Time PCR Analysis)

Total RNA was prepared with an RNeasy Mini Kit and an RNase-Free DNase (QIAGEN). First-strand cDNA was synthesized from the total RNA using a PrimeScript® II (Takara Bio), and a real-time PCR reaction was performed using a StepOnePlus Real-Time PCR System (Applied Biosystems, Foster City, CA) and an SYBR Premix Ex Taq II (Takara Bio). The level of target mRNA was determined by using the ΔΔCt method with a GAPDH used as an internal standard.

The primers (5'-3') used for quantitative real-time PCR analysis are as follows.

OCT4_F: GCTCGAGAAGGATGTGGTCC (SEQ ID NO: 13)
OCT4_R: CGTTGTGCATAGTCGCTGCT (SEQ ID NO: 14)
NANOG_F: GCAGAAGGCCTCAGCACCTA (SEQ ID NO: 15)
NANOG_R: AGGTTCCCAGTCGGGTTCA (SEQ ID NO: 16)
GATA2_F: TCAAGCCCAAGCGAAGAC (SEQ ID NO: 17)
GATA2_R: CACAGGCGTTGCAGACAG (SEQ ID NO: 18)
GATA3_F: CTCATTAAGCCCAAGCGAAG (SEQ ID NO: 19)
GATA3_R: TCTGACAGTTCGCACAGGAC (SEQ ID NO: 20)
TFAP2A_F: AACATGCTCCTGGCTACAAAA (SEQ ID NO: 21)
TFAP2A_R: AGGGGAGATCGGTCCTGA (SEQ ID NO: 22)
TP63_F: AGAAACGAAGATCCCCAGATGA (SEQ ID NO: 23)
TP63_R: CTGTTGCTGTTGCCTGTACGTT (SEQ ID NO: 24)

(Bisulfite Sequencing)

Genomic DNA was purified by phenol/chloroform extraction and ethanol precipitation. An EZ DNA Methylation-Gold Kit (Zymo Research, Orange, CA) was used for treatment of genomic DNA with bisulfite. A TaKaRa EpiTaq HS (Takara Bio) was used for PCR. PCR products were purified, and cloned into a pGEM-T vector (Promega). Sequencing of individual clones was performed by Eurofins Genomics (Tokyo, Japan). On average, 20 clones were sequenced for each sample.

The primers (5'-3') used for bisulfite sequencing are as follows.

ELF5_BSF: TGATGGATATTGAATTTGAATTTAAAGGTA (SEQ ID NO: 25)
ELF5_BSR: CAATAAAAATAAAAACACCTATAACCTTAT (SEQ ID NO: 26)

( Immunostaining)

Cells were fixed with 4% paraformaldehyde (Fujifilm Wako) for 10 minutes, and was subjected to 0.3% Triton X-100 (Fujifilm Wako) for 5 minutes for permeabilization, before blocking with 2% FBS/PBS at room temperature for 1 hour. The cells were then incubated overnight at 4°C with primary antibodies. Alexa Fluor 488 or Alexa Fluor 555-binding secondary antibodies (Cell Signaling) were used as secondary antibodies. The nucleus was stained with Hoechst 33258, and photographed with a fluorescence microscope (BZ-X710, Keyence, Osaka, Japan).

The primary antibodies used are as follows.

PE-conjugated anti-HLA-G (Novus Biologicals, Littleton, CO; 1:100 dilution),
PE-conjugated anti-SDCl (#130-119-840, Miltenyi Biotec, Bergisch Gladbach, Germany; 1:100 dilution)
Anti-GATA3 (#5852, Cell Signaling, Danvers, MA; 1:100 dilution)
Anti-TFAP2C (#sc-12762, Santa Cruz Biotechnology, Dallas, TX; 1:100 dilution)
Anti-hCG (#IS508, Dako, Hamburg, Germany; 1:10 dilution)
Anti-OCT4 (#4439, Cell Signaling; 1:100 dilution)

(Creation of Graphs)

The methylation level of CpG cytosine was visualized with Integrative Genomics Viewer (IGV) software (www.broadinstitute.org/igv/). Heat maps were crated using the heatmap.2 function of the gplots package. Bar graphs were created using the ggplot2 package available from R (www.R-project.org/).

[Results]

<TS-Like Cells Derived from iPS Cells>

(Preparation of TS-Like Cells from iPS Cells)

iPS cells were induced to differentiate into TS-like cells following the method described in the foregoing section (Induced Differentiation into TS-Like Cells via BMP4 Signaling). This produced TS-like cells similar in morphology to TS cells (see FIG. 1).

Separately, iPS cells were induced to differentiate into TS cells following the method described in the foregoing section (Induced Differentiation into TS-Like Cells by Gene Introduction). This produced TS-like cells similar in morphology to TS cells, irrespective of which of the GATA3, GATA2, and TFAP2A genes (cDNAs) was introduced (see FIG. 1). As a representative example, FIG. 1 shows TS-like cells obtained after introduction of GATA3 gene into iPS cells.

(Gene Expression Analysis)

Gene expression in TS-like cells obtained after introduction of GATA2, GATA3, or TFAP2A gene into iPS cells was analyzed, and the result was compared with that from TS cells.

The result of comparison is presented in FIG. 2. FIG. 2 shows expression levels relative to the expression level of iPS cells taken as log2(1). As shown in FIG. 2, the TS-like cells obtained after gene introduction had similar gene expression to TS cells, regardless of the gene. The TS-like cells were also similar to TS cells in that these cells both had reduced expression of OCT4 and NANOG, marker genes of iPS cells.

<TS-Like Cells Derived from ES Cells>

(Preparation of TS-Like Cells from ES Cells)

ES cells were induced to differentiate into TS-like cells following the method described in the foregoing section (Induced Differentiation into TS-Like Cells via BMP4 Signaling). This produced TS-like cells similar in morphology to TS cells.

( Immunostaining)

Anti-OCT4 antibodies (anti-OCT4), anti-TFAP2C antibodies (anti-TFAP2C), and anti-GATA3 antibodies (anti-GATA3) were used for immunostaining of TS-like cells, ES cells, and TS cells.

The results are presented in FIG. 3. In FIG.3 and subsequent figures, "ES-TS" represents TS-like cells derived from ES cells. As shown in FIG. 3, the TS-like cells derived from ES cells had gene expression patterns that were more similar to TS cells than to ES cells.

(Gene Expression Analysis)

Gene expression of TS-like cells was analyzed, and the result was compared with results from ES cells, BMP4-treated cells (cells after a BMP4 treatment of ES cells using a conventional technique; cultured in BMP4 medium for 72 hours), and TS cells.

The results are presented in FIG. 4A and FIG. 4B. In FIGS. 4A and 4B and subsequent figures, "ES-BMP4" represents cells after a BMP4 treatment of ES cells using a conventional technique. As shown in FIGS. 4A and 4B, the TS-like cells derived from ES cells had gene expression patterns that were more similar to TS cells than to ES cells. Specifically, the TS-like cells were negative for expression of NANOG, OCT4, and SOX2, and positive for expression of GATA3, GATA2, TFAP2C, TFAP2A, and TP63.

In contrast, the BMP4-treated cells prepared by using a conventional technique still showed high expression of transcription factors specific to ES cells. The BMP4-treated cells also had low expression of transcription factors specific to TS cells, compared to the TS-like cells and ES cells. The result for TP63, expressed in TS cells and TS-like cells, was negative in the BMP4-treated cells prepared by using a conventional technique, as in ES cells.

FIG. 5 is a diagram representing correlations of gene expression between TS cells, TS-like cells (ES-TS), ES cells, and ES cells subjected to a BMP4 treatment using a conventional technique (ES-BMP4 cells). It can be seen that TS-like cells and TS cells have a high correlation. The BMP4-treated cells prepared by using a conventional technique had a higher correlation with ES cells than with TS cells.

(Analysis of DNA Methylation Pattern)

The DNA methylation pattern of TS-like cells was analyzed, and the result was compared with results from ES cells, BMP4-treated cells (cells after a BMP4 treatment of ES cells using a conventional technique), and TS cells.

The result of comparison is presented in FIG. 6. FIG. 6 shows analysis results for a 10 Mb region in chromosome 21. As shown in FIG. 6, the TS-like cells derived from ES cells had a DNA methylation pattern that was more similar to TS cells than to ES cells. In contrast to the ES cells in which the percentage of methylation in the whole genome was about 81%, the percentage of methylation in TS-like cells was reduced to about 45%, about the same as the result observed for TS cells.

The BMP4-treated cells prepared by using a conventional technique had a methylation pattern similar to that of ES cells.

(DNA Methylation Analysis for ELF5)

A DNA methylation analysis was performed for TS-like cells, ES cells, and TS cells in the promoter region (chr11: 34513753-34514270 (transcription start site: chr11: 34513800)) of ELF5 gene. ELF5 is a gene that has been reported to show low methylation in the placenta (Hemberger M, et al., Hum Mol Genet. 2010 Jun 15;19(12):2456-67).

The results are presented in FIG. 7. As shown in FIG. 7, the TS-like cells had a percentage methylation of 1.0%, similar to the percentage of methylation observed for the TS cells.

A previous study reports that the percentage of ELF5 methylation in cells prepared by a conventional BMP4 treatment of ES cells is 29.5% (Lee CQ, et al., Stem Cell Rep. 2016 6:257-272). It therefore can be seen that, with regard to a fraction of DNA methylation in ELF5, the TS-like cells are far more similar to TS cells than BMP4-treated cells prepared by using a conventional technique.

(Differentiation Test for TS-Like Cells)

TS-like cells and TS cells were tested to differentiate into EVTs or STs, following the method described in the foregoing section (Differentiation into EVTs or STs). The differentiated cells were analyzed for expression of HLA-G and hCG, which are markers of EVT and ST, respectively.

The results of differentiation test for EVT are presented in FIG. 8A and FIG. 8B. FIG. 8A shows the result of immunostaining with anti-HLA-G antibodies (PE-conjugated anti-HLA-G) performed for cells differentiated into EVTs from TS-like cells. FIG. 8B shows the result of comparison of HLA-G expression levels between each cell type. In FIG. 8B (and FIG. 9B), "ES-TS_EVT" represents cells differentiated into EVTs from TS-like cells, and "TS_EVT" represents cells differentiated into EVTs from TS cells.

As shown in FIGS. 8A and 8B, the cells differentiated into EVTs from TS-like cells had increased expression of HLA-G, as in the cells differentiated into EVTs from TS cells. This result confirmed that the TS-like cells have the potential to differentiate into EVTs, as does the TS cells.

The results of differentiation test for ST are presented in FIG. 9A and FIG. 9B. FIG. 9A shows the result of immunostaining with anti-hCG antibodies (anti-hCG) performed for cells differentiated into STs from TS-like cells. FIG. 9B shows the result of comparison of hCG expression levels between each cell type.

As shown in FIGS. 9A and 9B, the cells differentiated into STs from TS-like cells had increased expression of hCG, as in the cells differentiated into STs from TS cells. This result confirmed that the TS-like cells have the potential to differentiate into STs, as does the TS cells.

(DNA Methylation Analysis for DNMT1 and ZFAT)

A DNA methylation analysis was performed for TS-like cells, ES cells, and TS cells in the promoter region of DNMT1 gene (transcription start site of DNMT1 gene (chr19: 10192830-10195739; transcription start site: chr19: 10195054)) and the promoter region of ZFAT gene (chr8: 134694984-134697871; transcription start site: chr8: 134696558)). These regions are known to show placenta-specific methylation (germline differentially methylated regions: gDMRs).

The results are presented in FIG. 10. As shown in FIG. 10, the TS-like cells had a lower percentage of methylation than the TS cells in DMNT1 gene. The TS-like cells had a higher percentage of methylation than the TS cells in ZFAT gene. These results indicate that the TS-like cells have different characteristics from TS cells with regard to the methylation patterns of these genes.

(Gene Expression Analysis for BRDT and SOHLH2)

A gene expression analysis was performed for BRDT gene and SOHLH2 gene in TS-like cells, TS cells, and ES cells.

The result is presented in FIG. 11. As shown in FIG. 11, the TS-like cells, unlike TS cells, showed hardly any expression of BRDT and SOHLH2 genes. This result indicates that the TS-like cells have different characteristics from TS cells with regard to the expression of these genes.

Industrial Applicability

According to the present invention, a method for producing a TS-like cell from a pluripotent stem cell is provided. The present invention also provides a TS-like cell produced by the method. TS-like cells provided by the present invention are applicable to studies of early development of mammals, and functional analyses of the placenta. Other applicable areas include regenerative medicine.

## Claims

1. A cell derived from a pluripotent stem cell of a mammal and that is capable of differentiating into a placenta-forming cell, and that is negative for BRDT, and positive for TP63.

2. The cell according to claim 1, which is negative for at least one selected from the group consisting of Oct4, Nanog, and Sox2.

3. The cell according to claim 1 or 2, which is positive for at least one selected from the group consisting of GATA2, GATA3, and TFAP2A.

4. The cell according to any one of claims 1 to 3, wherein a fraction of methylation of genomic DNA is 60% or less of whole genome.

5. The cell according to any one of claims 1 to 4, wherein the placenta-forming cell is an extravillous cytotrophoblast or a syncytiotrophoblast.

6. A method for producing a cell capable of differentiating into a placenta-forming cell,
the method comprising the steps of:

   (a) culturing a pluripotent stem cell of a mammal with a medium containing bone morphogenetic protein 4; and
   (b) culturing the cell after the step (a) with a medium containing a growth factor and a ROCK inhibitor.

7. A method for producing a cell capable of differentiating into a placenta-forming cell,
the method comprising the steps of:

   (a') introducing at least one gene selected from the group consisting of GATA2, GATA3, and TFAP2A to a pluripotent stem cell of a mammal; and
   (b) culturing the cell after the step (a') with a medium containing a growth factor and a ROCK inhibitor.

8. The method for producing a cell capable of differentiating into a placenta-forming cell according to claim 6 or 7, wherein the medium in the step (b) further contains at least one selected from the group consisting of an ALK5 inhibitor and a GSK3β inhibitor.

FIG. 1

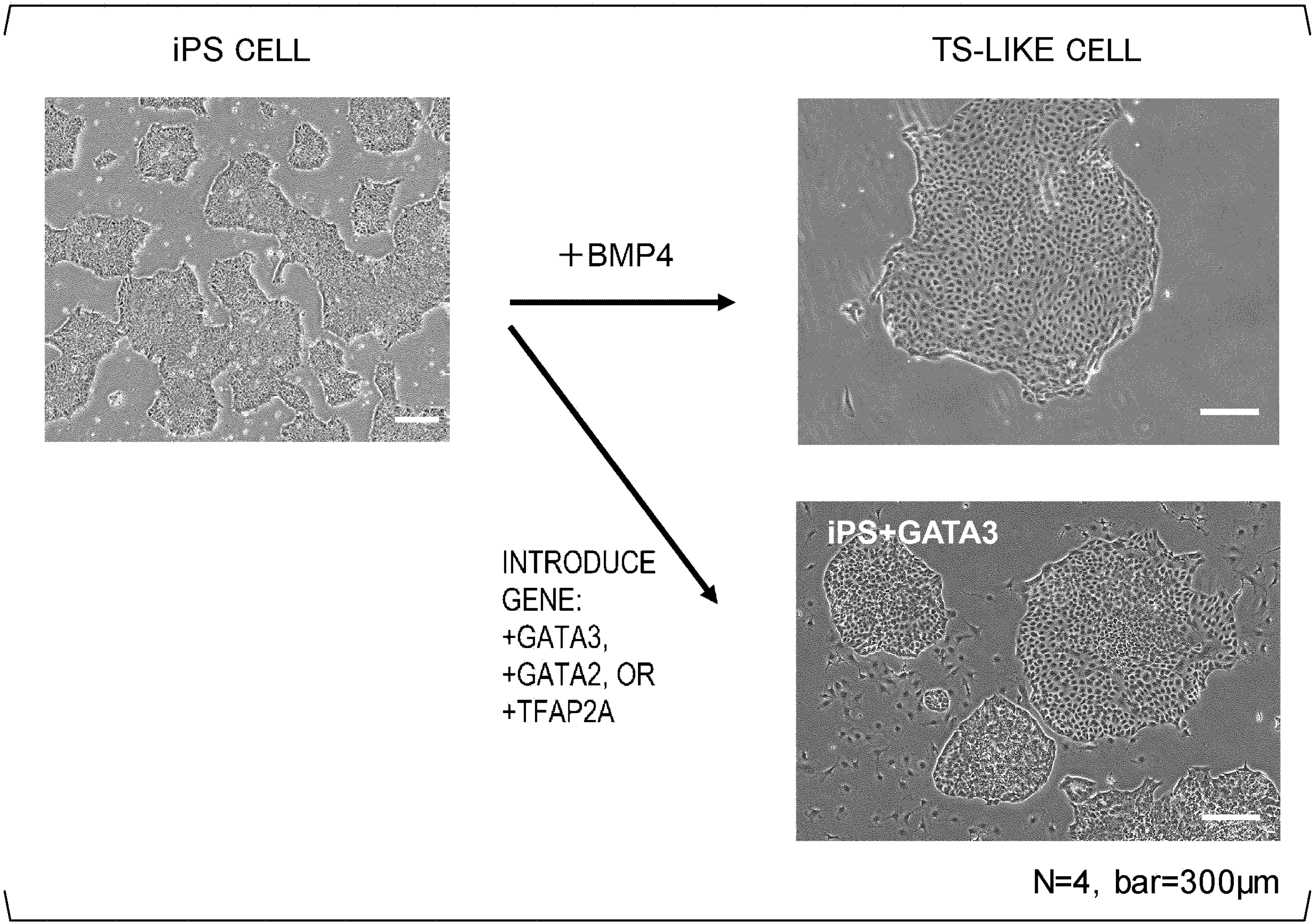
iPS CELL
TS-LIKE CELL
+BMP4
INTRODUCE GENE: +GATA3, +GATA2, OR +TFAP2A
iPS+GATA3
N=4, bar=300μm

FIG. 2

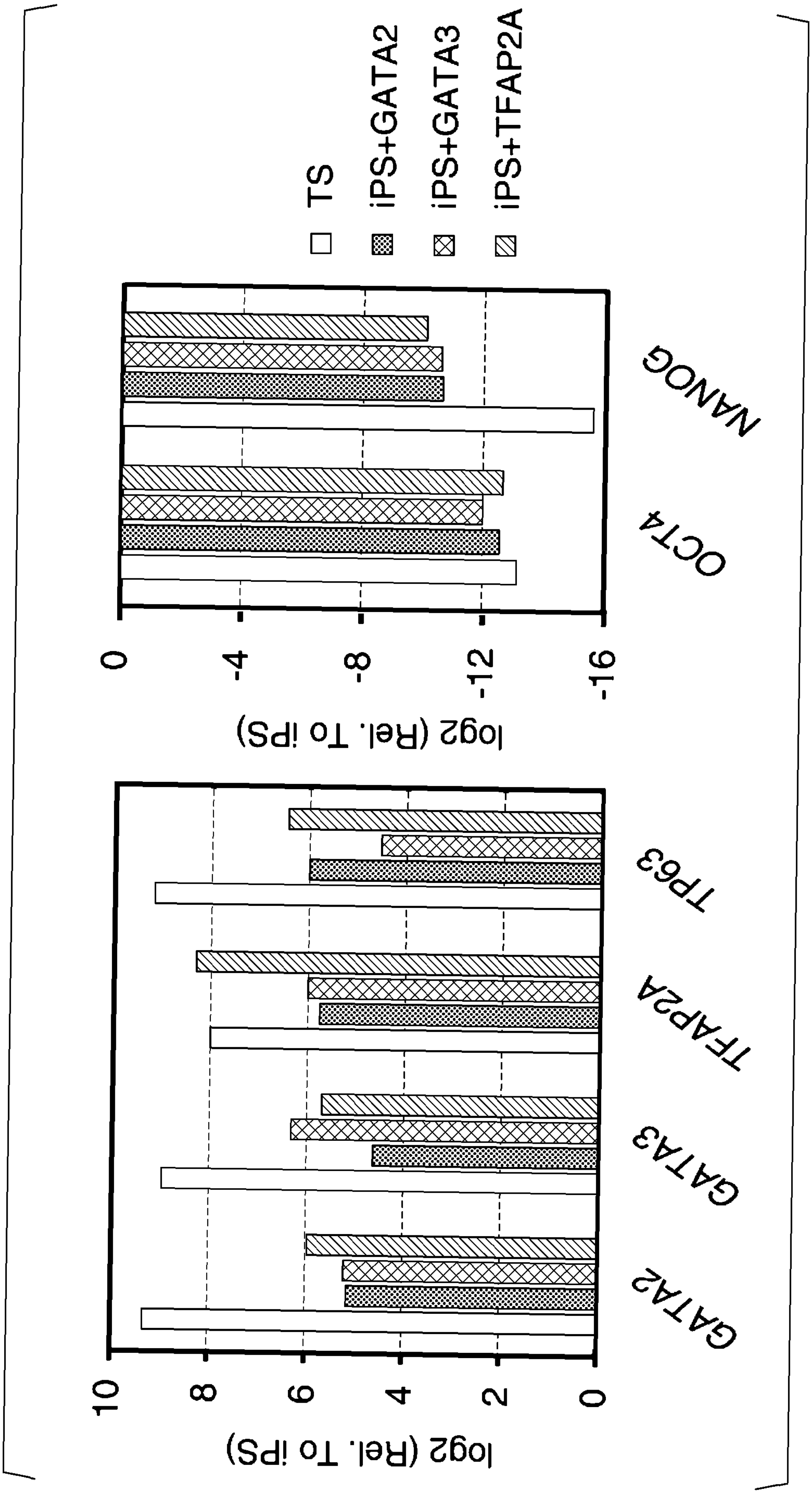
log2 (Rel. To iPS)
10
8
6
4
2
0
GATA2
GATA3
TFAP2A
TP63
log2 (Rel. To iPS)
0
-4
-8
-12
-16
OCT4
NANOG
TS
iPS+GATA2
iPS+GATA3
iPS+TFAP2A

FIG. 3

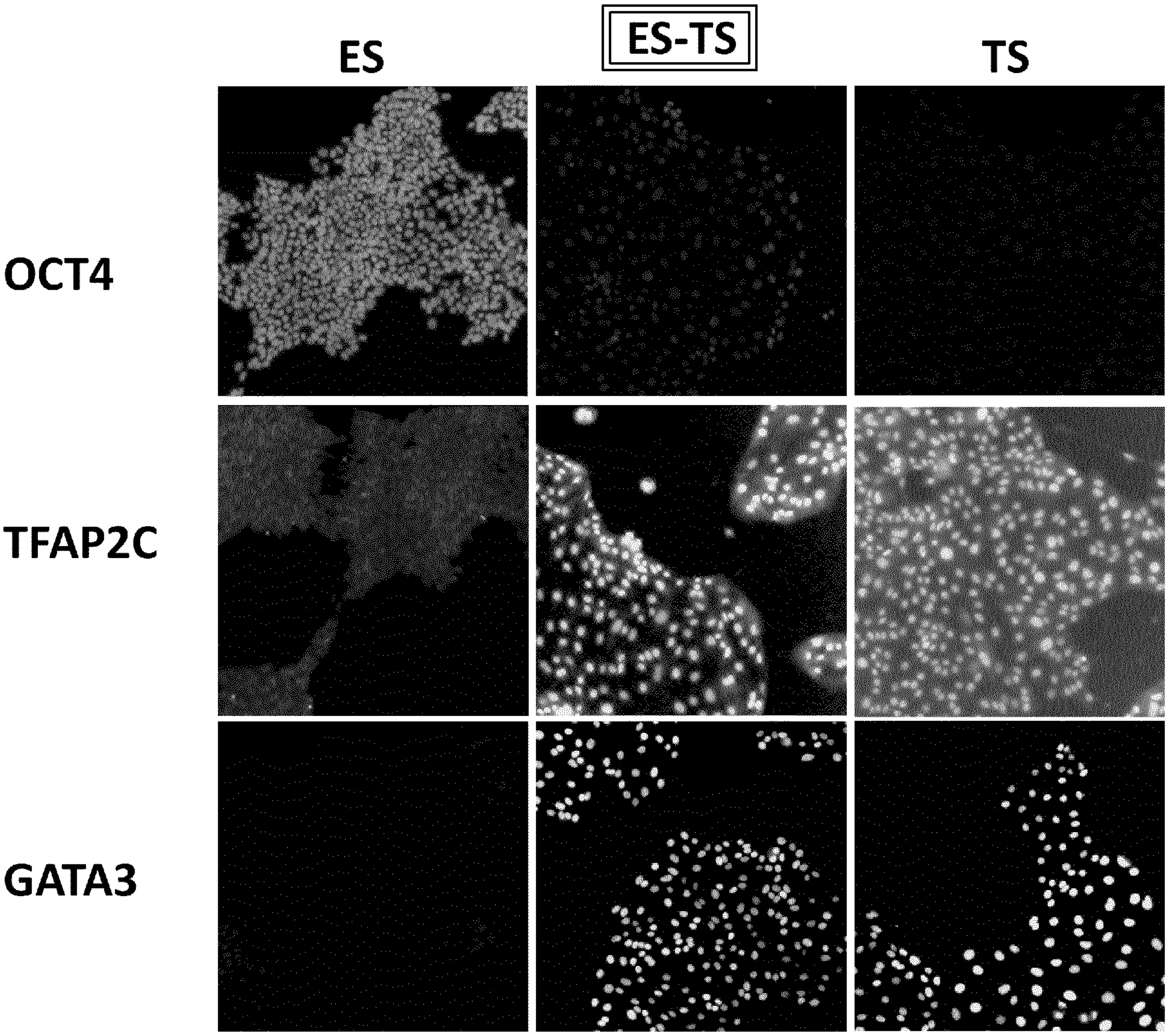

FIG. 4A

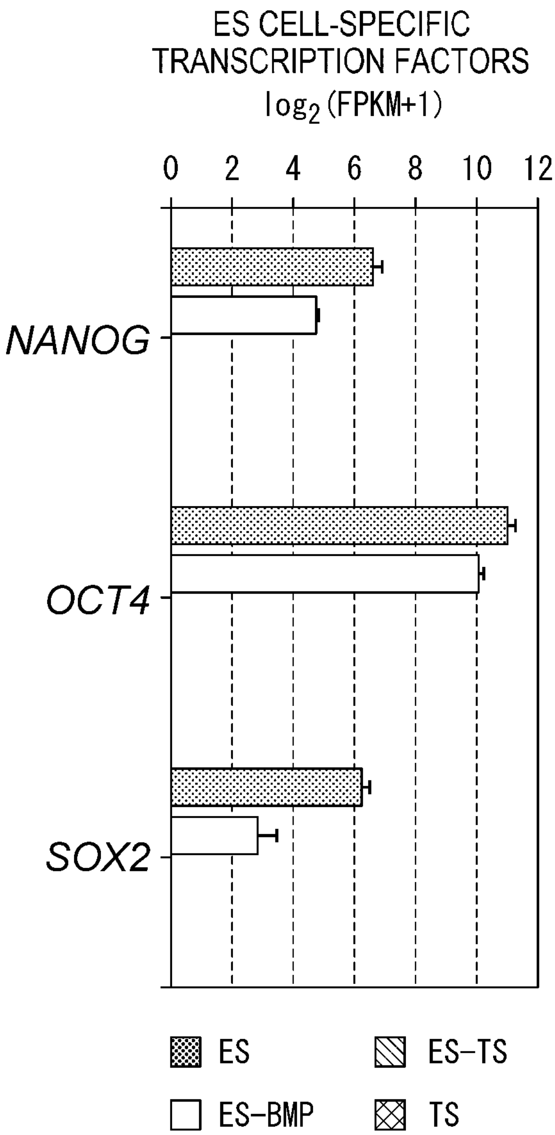
ES CELL-SPECIFIC
TRANSCRIPTION FACTORS
log2 (FPKM+1)
0
2
4
6
8
10
12
NANOG
OCT4
SOX2
ES
ES-TS
ES-BMP
TS

# FIG. 4B

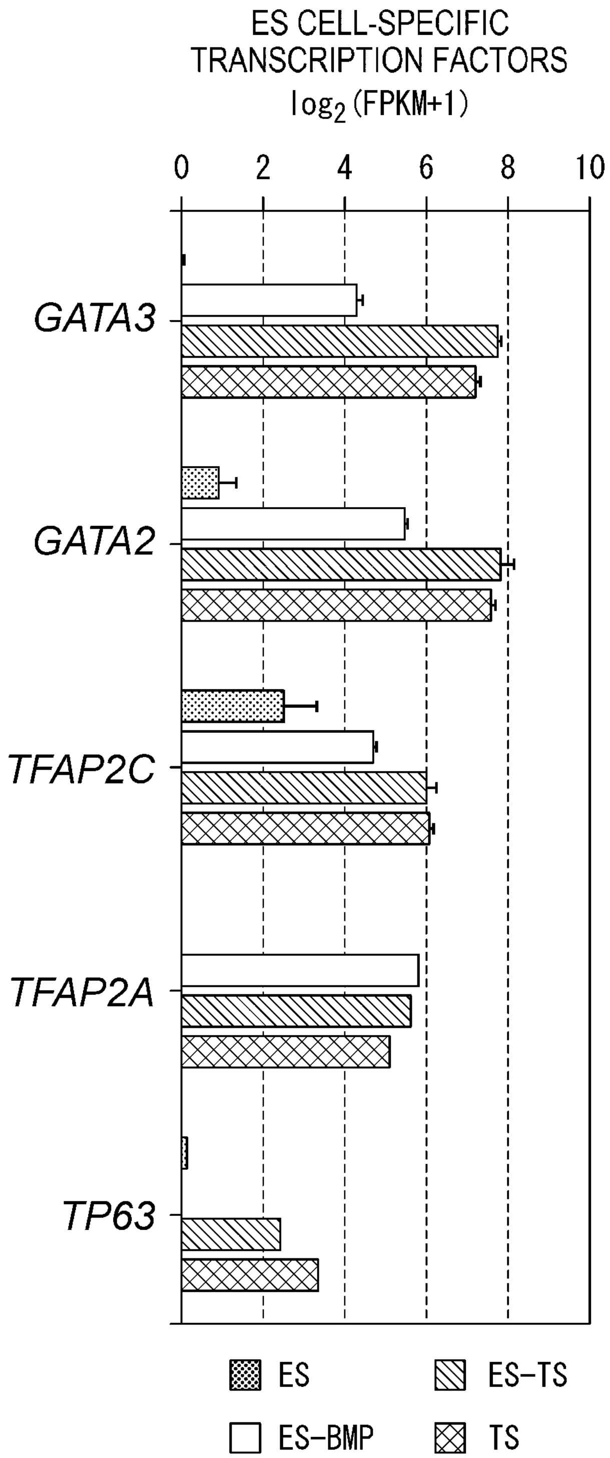
ES CELL-SPECIFIC
TRANSCRIPTION FACTORS
$\log_2$(FPKM+1)
0
2
4
6
8
10
GATA3
GATA2
TFAP2C
TFAP2A
TP63
ES
ES-BMP
ES-TS
TS

FIG. 5

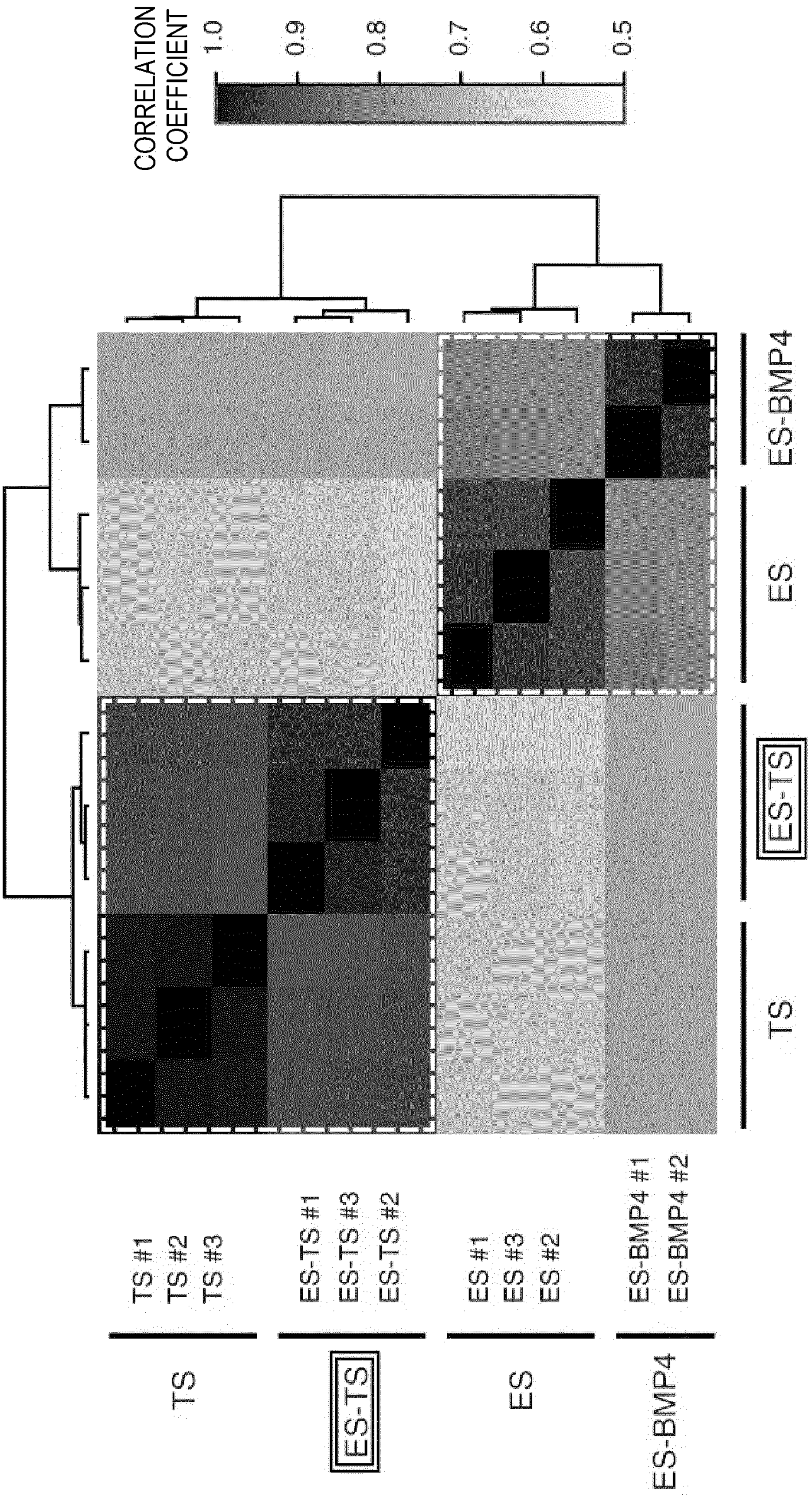
CORRELATION COEFFICIENT
1.0
0.9
0.8
0.7
0.6
0.5
TS
TS #1
TS #2
TS #3
ES-TS
ES-TS #1
ES-TS #3
ES-TS #2
ES
ES #1
ES #3
ES #2
ES-BMP4
ES-BMP4 #1
ES-BMP4 #2
TS
ES-TS
ES
ES-BMP4

# FIG. 6

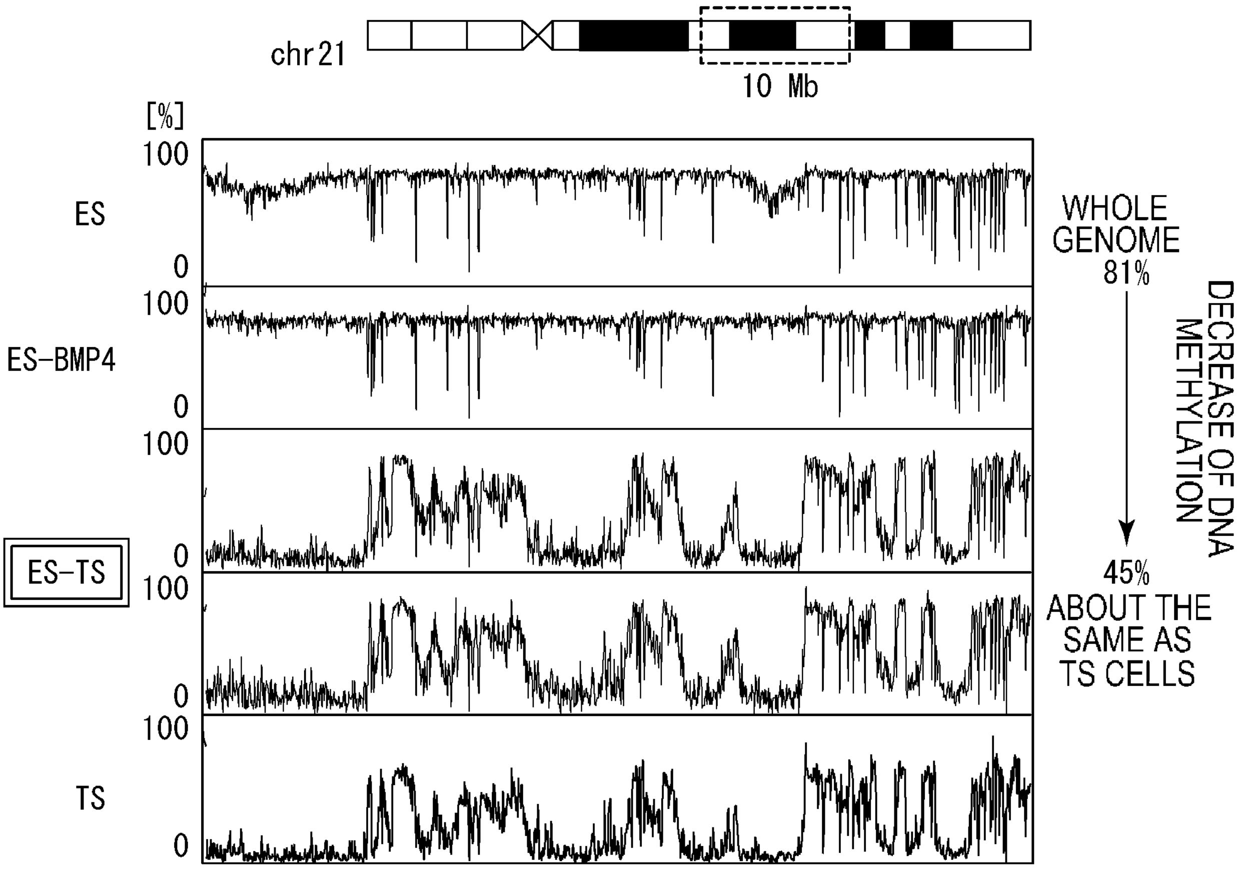
chr21
10 Mb
[%]
100
0
ES
ES-BMP4
ES-TS
TS
WHOLE GENOME 81%
DECREASE OF DNA METHYLATION
45%
ABOUT THE SAME AS TS CELLS

FIG. 7

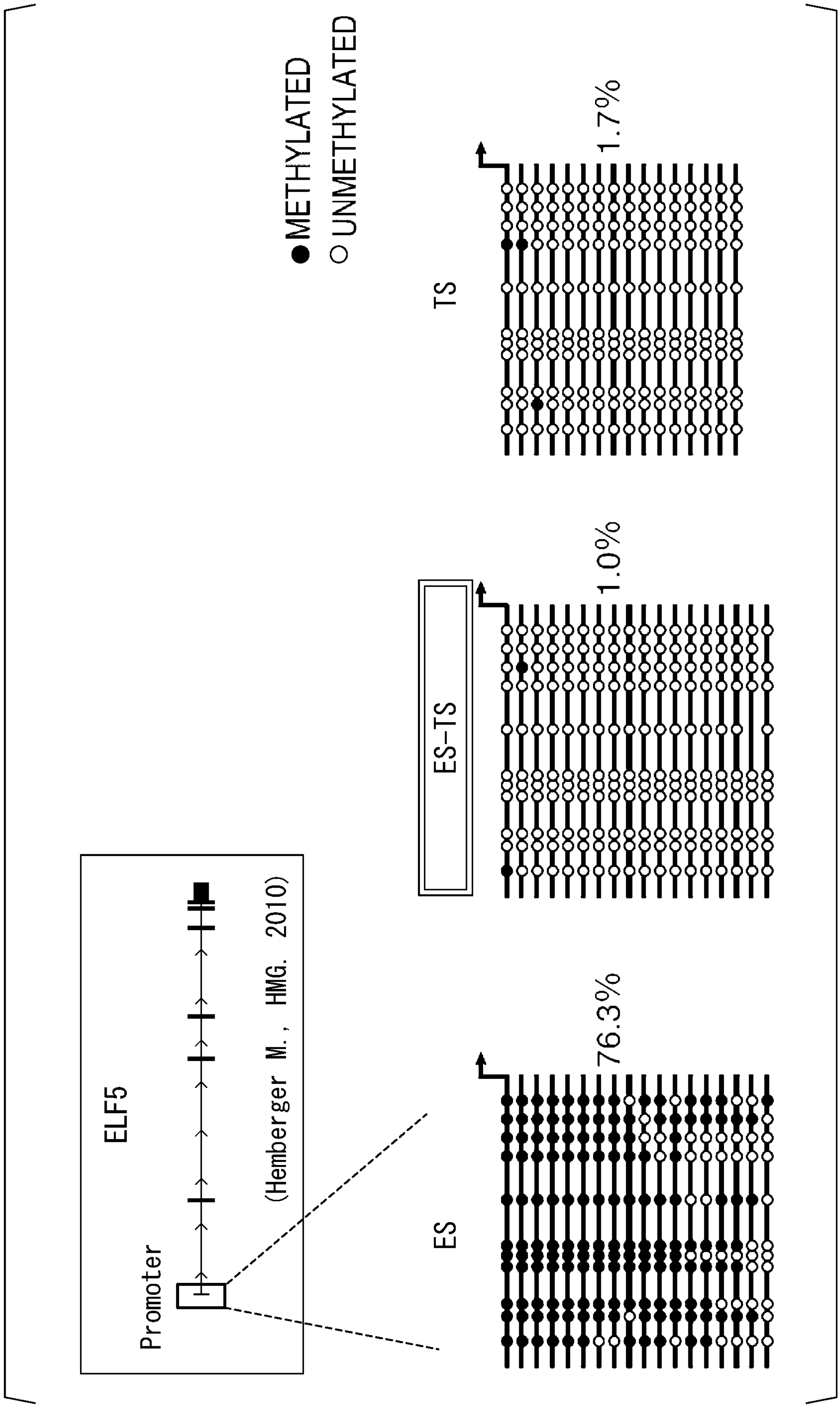

ELF5
Promoter
(Hemberger M., HMG. 2010)
ES
76.3%
ES-TS
1.0%
TS
1.7%
●METHYLATED
○UNMETHYLATED

FIG. 8A

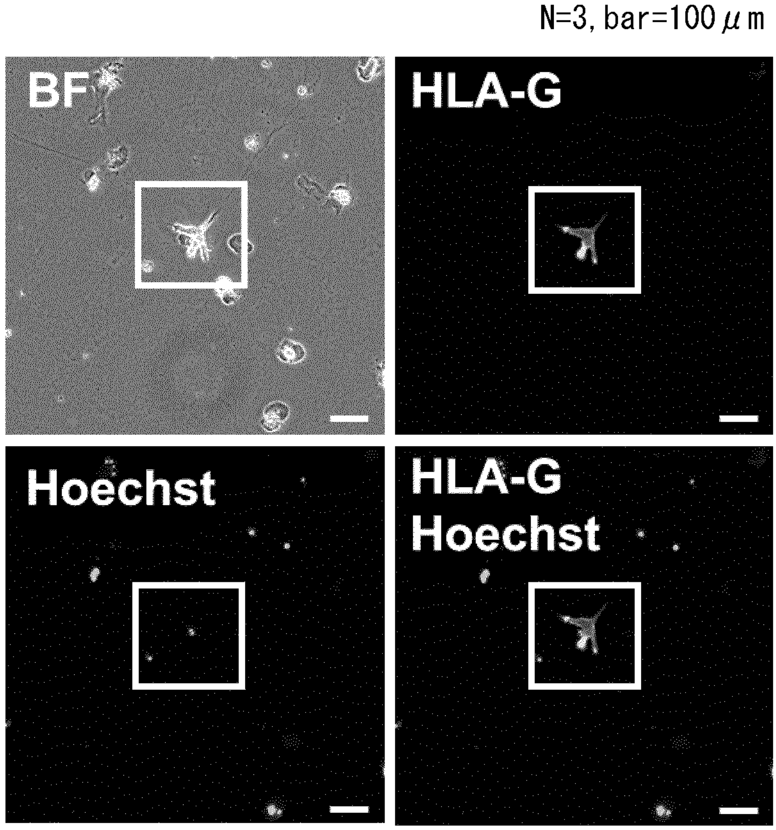
N=3, bar=100 μm
BF
HLA-G
Hoechst
HLA-G
Hoechst

FIG. 8B

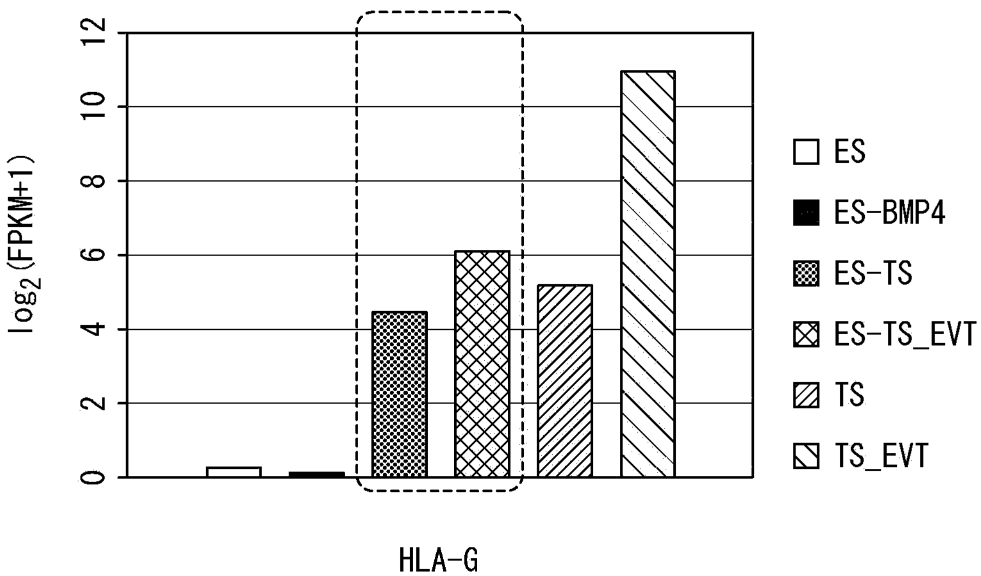
log2(FPKM+1)
0
2
4
6
8
10
12
HLA-G
ES
ES-BMP4
ES-TS
ES-TS_EVT
TS
TS_EVT

FIG. 9A

N=3, bar=100 μm

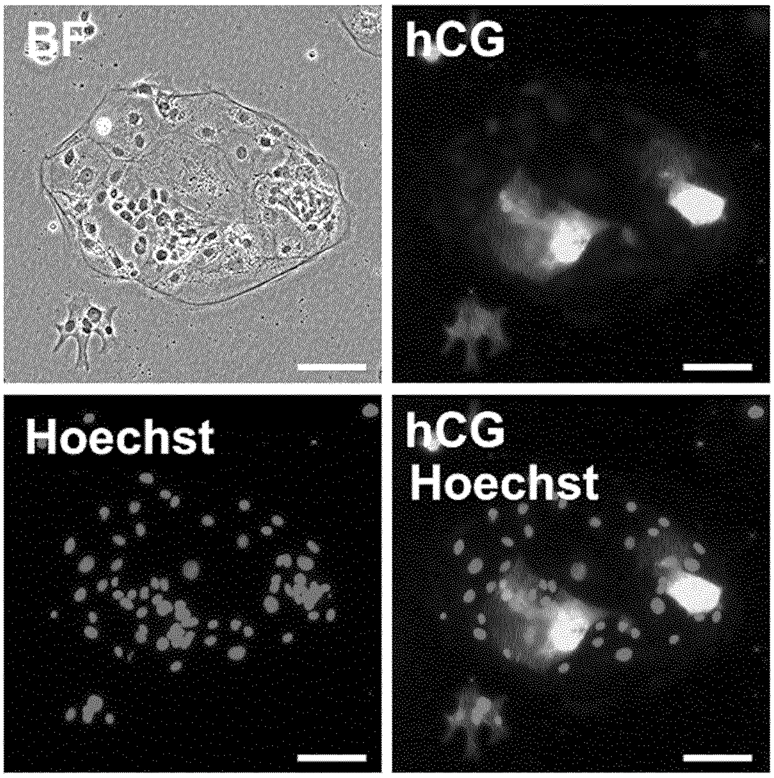


FIG. 9B

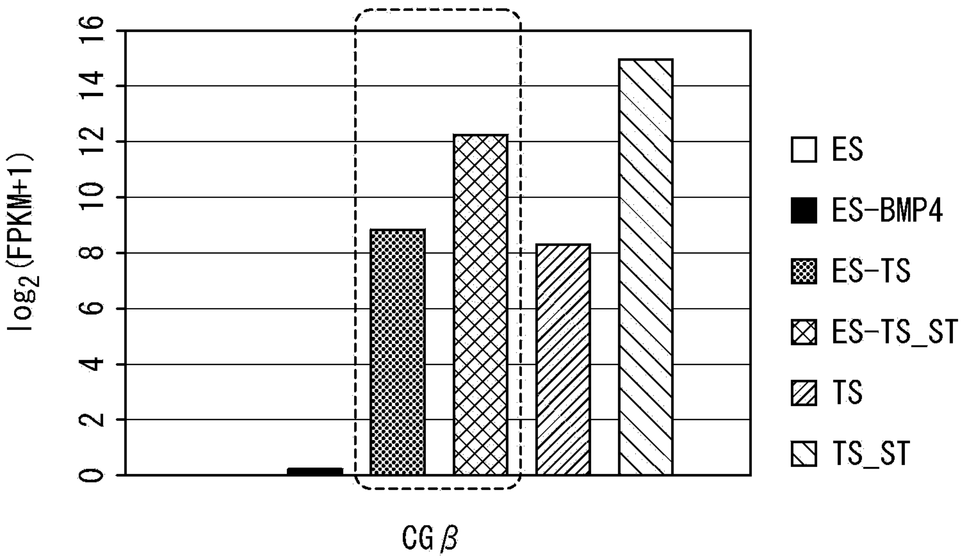

FIG. 10

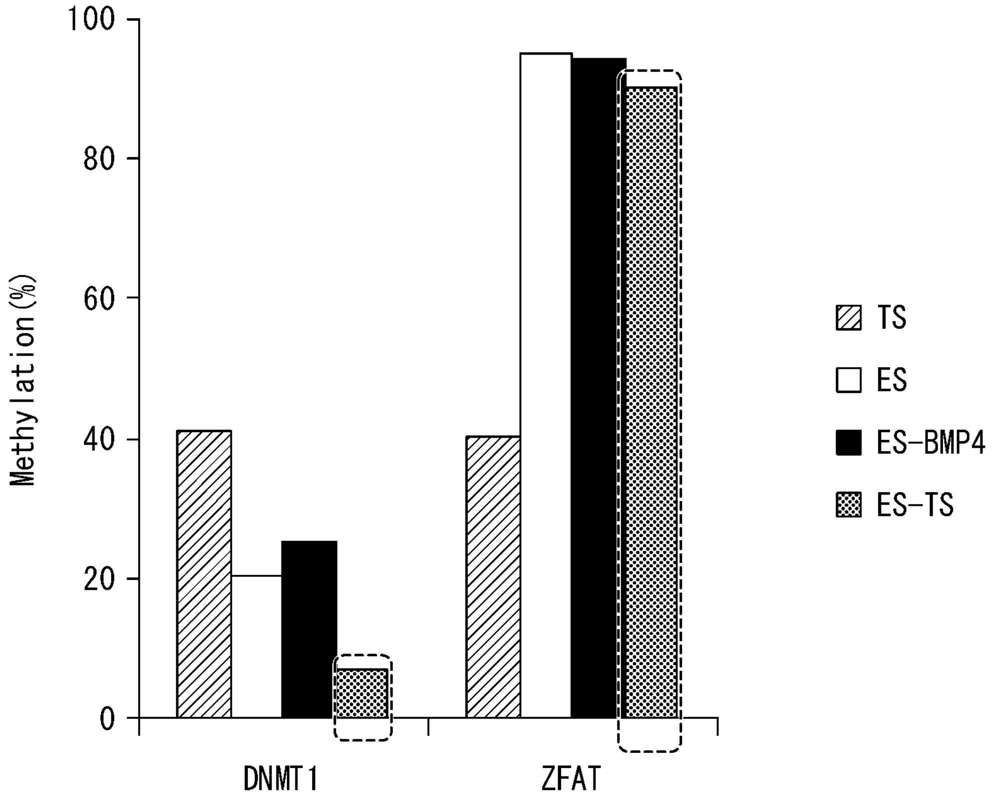
100
80
60
40
20
0
Methylation (%)
DNMT1
ZFAT
TS
ES
ES-BMP4
ES-TS

FIG. 11

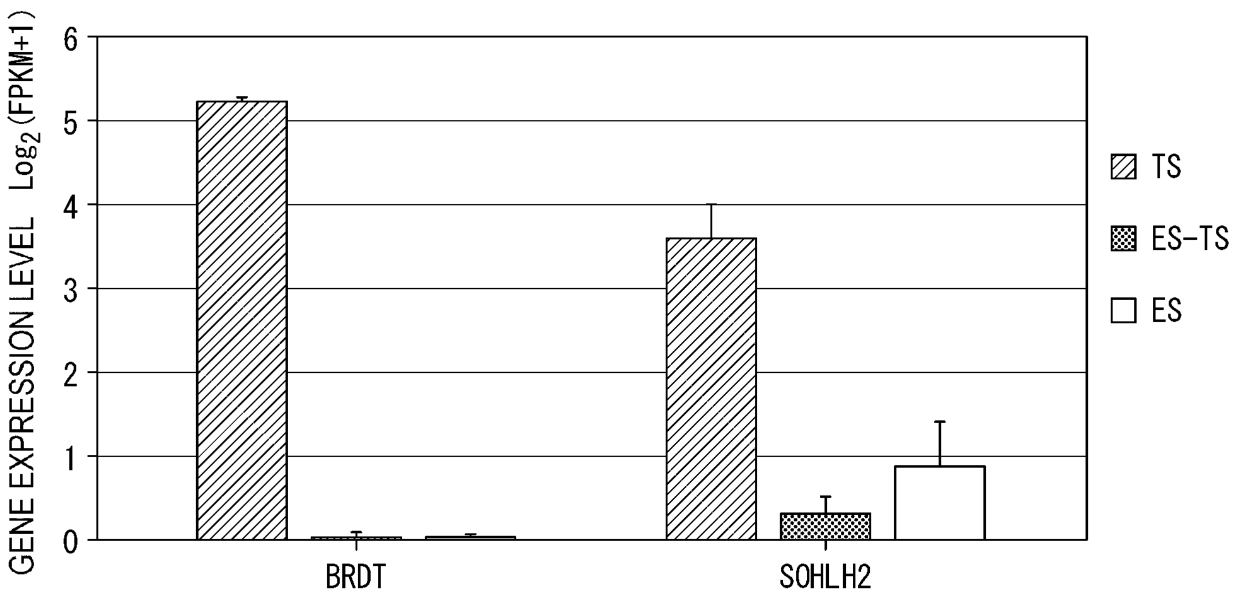
6
5
4
3
2
1
0
GENE EXPRESSION LEVEL Log2 (FPKM+1)
BRDT
SOHLH2
TS
ES-TS
ES

## INTERNATIONAL SEARCH REPORT

International application No. PCT/JP2019/023725

A. CLASSIFICATION OF SUBJECT MATTER
Int. Cl. C12N5/073(2010.01)i, C12N15/09(2006.01)i

According to International Patent Classification (IPC) or to both national classification and IPC

B. FIELDS SEARCHED

Minimum documentation searched (classification system followed by classification symbols)
Int. Cl. C12N5/073, C12N15/09

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
JSTPlus/JMEDPlus/JST7580 (JDreamIII), CAplus/MEDLINE/EMBASE/BIOSIS/STN (STN), CiNii, PubMed

C. DOCUMENTS CONSIDERED TO BE RELEVANT

| Category* | Citation of document, with indication, where appropriate, of the relevant passages | Relevant to claim No. |
|---|---|---|
| Y | KRENDL, C. et al., GATA2/3 - TFAP2A/C transcription factor network couples human pluripotent stem cell differentiation to trophectoderm with repression of pluripotentcy., Proc. Natl. Acad. Sci. USA, 2017, 114(45), E9579-E9588, Supporting Information, abstract, page E9579, right column, fourth line from the bottom to page E9580, left column, line 2, page E9580, left column, nineteenth line from the bottom to page E9581, right column, fourth line from the bottom, page E9584, right column, line 10 to page E9586, right column, line 18, Supporting Information "Trophoblast Differentiation", fig. 1, 2E, 3, 5G, H | 1-8 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 22.08.2019 | 03.09.2019 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2019/023725

C (Continuation). DOCUMENTS CONSIDERED TO BE RELEVANT

| Category* | Citation of document, with indication, where appropriate, of the relevant passages | Relevant to claim No. |
|---|---|---|
| Y | OKAE, H. et al., Derivation of Human Trophoblast Stem Cells., Cell Stem Cell, 2018, 22, pp. 50-63, e1-e6, abstract, page 52, left column, line 24 to page 54, right column, line 12, page 56, right column, lines 3-7, fig. 1-5、page e4 "Differentiation of TSCT and TSblast cells" | 1-8 |
| Y | WO 2016/143866 A1 (TOHOKU UNIVERSITY) 15 September 2016, claims, in particular, claim 8, examples (Family: none) | 1-8 |
| Y | GAMAGE, T. et al., Stem cell insights into human trophoblast lineage differentiation., Human Reproduction Update, 2017, vol. 23, no. 1, pp. 77-103, tables 1, 2 | 1-8 |
| A | WO 2016/186078 A1 (SUMITOMO CHEMICAL COMPANY, LIMITED) 24 November 2016 & US 2018/0135022 A1 & EP 3299450 A1 | 1-8 |
| A | JP 2005-520514 A (WICELL RESEARCH INSTITUTE, INC.) 14 July 2005 & US 2004/0005701 A1 & WO 2003/078599 A2 & EP 1490506 A2 | 1-8 |

Form PCT/ISA/210 (continuation of second sheet) (January 2015)

**INTERNATIONAL SEARCH REPORT**

International application No.
PCT/JP2019/023725

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

(see extra sheet)

1. ☐ As all required additional search fees were timely paid by the applicant, this international search report covers all searchable claims.
2. ☒ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**

International application No.
PCT/JP2019/023725

(continuation of Box No. III)

The claims are classified as the following three inventions.

(Invention 1) Claims 1-5

Claims 1-5 are classified as invention 1 due to having the special technical feature of "cells derived from pluripotent stem cells of a mammal, having an ability to differentiate into cells for forming placenta, wherein BRDT is negative, and TP63 is positive."

(Invention 2) Claims 6, 8

Claim 6 shares, with claim 1 classified as invention 1, the feature of "cells having an ability to differentiate into cells for forming placenta." However, as disclosed in documents 1-3 cited in the ISR, trophoblast stem cells (TS cells) or TS-like cells having an ability to differentiate into cells for forming placenta are well-known in the corresponding field, and thus this feature does not make a contribution over the prior art and cannot be said to be a special technical feature. Furthermore, there are no other identical or corresponding special technical features between claim 6 and claim 1.

Moreover, claim 6 is not a dependent claim of claim 1. Additionally, claim 6 is not substantially identical to or similarly closely related to any of the claims classified as invention 1.

Therefore, claim 6 cannot be classified as invention 1. The same also applies to claim 8 citing claim 6.

(Invention 3) Claims 7, 8

Claim 7 shares, with claim 1 classified as invention 1, the feature of "cells having an ability to differentiate into cells for forming placenta."

Form PCT/ISA/210 (extra sheet) (January 2015)

INTERNATIONAL SEARCH REPORT

International application No.
PCT/JP2019/023725

However, as described above, trophoblast stem cells (TS cells) or TS-like cells having an ability to differentiate into cells for forming placenta are well-known in the corresponding field, and thus this feature does not make a contribution over the prior art and cannot be said to be a special technical feature. Furthermore, there are no other identical or corresponding special technical features between claim 6 and claims 1 and 2.

Moreover, claim 7 is not a dependent claim of claim 1 or 6. Additionally, claim 7 is not substantially identical to or similarly closely related to any of the claims identified as invention 1 or invention 2.

Therefore, claim 7 cannot be classified as invention 1. The same also applies to claim 8 citing claim 7.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 6400832 B


### Non-patent literature cited in the description


- **OKAE H et al.** Derivation of Human Trophoblast Stem Cells. *Cell Stem Cell,* January 2018, vol. 22, 50-63
- **GAMAGE TK et al.** Stem cell insights into human trophoblast lineage differentiation. *Hum Reprod Update,* December 2016, vol. 23 (1), 77-103
- **SAMBROOK et al.** Molecular Cloning - A LABORATORY MANUAL. Cold Spring Harbor Laboratory Press
- **OKAE, H. ; TOH, H. ; SATO, T. et al.** *Cell Stem Cell,* 2018, vol. 22 (50-63), e56
- **KRENDL, C. ; SHAPOSHNIKOV, D. ; RISHKO, V. et al.** *Proc Natl Acad Sci USA,* 2017, vol. 114, E9579-E9588
- **TRAPNELL, C. ; ROBERTS, A. ; GOFF, L. et al.** *Nat Protoc,* 2012, vol. 7, 562-578
- **MIURA, F. ; ENOMOTO, Y. ; DAIRIKI, R. et al.** *Nucleic Acids Res,* 2012, vol. 40, e136
- **KRUEGER, F. ; ANDREWS, S. R.** *Bismark: Bioinformatics,* 2011, vol. 27, 1571-1572
- **HEMBERGER M et al.** *Hum Mol Genet.,* 15 June 2010, vol. 19 (12), 2456-67
- **LEE CQ et al.** *Stem Cell Rep.,* 2016, vol. 6, 257-272